# EUROPEAN PATENT APPLICATION

(11) **EP 2 266 542 A2**
(43) Date of publication of application: **29.12.2010**
(21) Application number: 10179341.2
(22) Date of filing: 01.10.1999
(51) Int. Cl.: A61K 9/26, A61K 9/24, A61K 9/50, A61K 9/32

(54) **Controlled release nanoparticulate compositions**

(30) Priority: 01.10.1998 US 164351; 22.06.1999 US 337675
(62) Divisional of application: 99950105.9
(71) Applicant: Elan Pharma International Limited, Monksland, Athlone Westmeath (IE)
(72) Inventor: Swanson, Jon, North Wales, PA 19454 (US); Jain, Rajeev, A, Norristown, PA 19401 (US); Hontz, Robert, Newtown Square, PA 19073 (US); Devane, John, G., Athlone (IE); Cumming, Kenneth, Iain, Phibsboro Dublin 7 (IE); Clancy, Maurice, Joseph, Anthony, Booterstown, Dublin (IE); Codd, Janet, Elizabeth, Athlone, County Westmeath (IE)
(74) Representative: Pohlman, Sandra M.

(57) **Abstract**

Described are controlled release nanoparticulate formulations comprising a nanoparticulate agent to be administered and a rate-controlling polymer which functions to prolong the release of the agent following administration. The novel compositions release the agent following administration for a time period ranging from about two to about twenty four hours or longer.

## Description

### FIELD OF THE INVENTION

The present invention relates to controlled release compositions containing a poorly soluble agent such as a drug. In particular, the present invention relates to compositions in which the poorly soluble agent is present in nanoparticulate form. The present invention also relates to solid oral dosage forms containing such compositions.

### BACKGROUND OF THE INVENTION

Controlled release refers to the release of an agent such as a drug from a composition or dosage form in which the agent is released according to a desired profile over an extended period of time. Controlled release profiles include, for example, sustained release, prolonged release, pulsatile release, and delayed release profiles. In contrast to immediate release compositions, controlled release compositions allow delivery of an agent to a subject over an extended period of time according to a predetermined profile. Such release rates can provide therapeutically effective levels of agent for an extended period of time and thereby provide a longer period of pharmacologic or diagnostic response as compared to conventional rapid release dosage forms. Such longer periods of response provide for many inherent benefits that are not achieved with the corresponding short acting, immediate release preparations. For example, in the treatment of chronic pain, controlled release formulations are often highly preferred over conventional short-acting formulations.

Controlled release pharmaceutical compositions and dosage forms are designed to improve the delivery profile of agents, such as drugs, medicaments, active agents, diagnostic agents, or any substance to be internally administered to an animal, including humans. A controlled release composition is typically used to improve the effects of administered substances by optimizing the kinetics of delivery, thereby increasing bioavailability, convenience, and patient compliance, as well as minimizing side effects associated with inappropriate immediate release rates such as a high initial release rate and, if undesired, uneven blood or tissue levels.

The term bioavailability is used to describe the degree to which a drug becomes available at the site(s) of action after administration. The degree and timing in which an agent such as a drug becomes available to the target site(s) after administration is determined by many factors, including the dosage form and various properties, *e.g.,* dissolution rate of the drug. It is well known that some drug compositions suffer from poor bioavailability because of poor solubility of the active ingredient itself.

Numerous methods have been developed for enhancing the bioavailability of poorly soluble drugs. Particle size reduction, such as nanoparticulate forms of the agent, is one such method since the dissolution rate of a compound is related to the particle size. Nanoparticulate compositions comprise poorly water-soluble drug or agent particles having an extremely small particle size, *i.e.,* less than one micron. With a decrease in particle size, and a consequent increase in surface area, a composition tends to be rapidly dissolved and absorbed following administration. For certain formulations, this characteristic can be highly desirable, as described, for example, in U.S. Patent Nos. 5,145,684, 5,510,118, 5,534,270, and 4,826,689, which are specifically incorporated by reference. However, rapid dissolution is contrary to the goal of controlled release. Known controlled release formulations do not present a solution to this problem.

Prior art teachings of the preparation and use of compositions providing for controlled release of an active compound provide various methods of extending the release of a drug following administration. However, none of the methods suggest a successful method of administering a nanoparticulate formulation.

Exemplary controlled release formulations known in the art include specially coated pellets, microparticles, implants, tablets, minitabs, and capsules in which a controlled release of a drug is brought about, for example, through selective breakdown of the coating of the preparation, through release through the coating, through compounding with a special matrix to affect the release of a drug, or through a combination of these techniques. Some controlled release formulations provide for pulsatile release of a single dose of an active compound at predetermined periods after administration.

U.S. Patent No. 5,110,605 to Acharya et al. refers to a calcium polycarbophil-alginate controlled release composition. U.S. Patent No. 5,215,758 to Krishnamurthy et al. refers to a controlled release suppository composition of sodium alginate and calcium salt. U.S. Patent No. 5,811,388 to Friend et al. refers to a solid alginate-based formulation including alginate, a water-swellable polymer, and a digestible hydrocarbon derivative for providing controlled release of orally administered compounds.

WO 91/13612 refers to the sustained release ofpharmaceuticals using compositions in which the drug is complexed with an ion-exchange resin. The specific ion-exchange resin described in this published patent application is AMBERLITE IRP 69^{®}, a sodium polystyrene sulphonate resin.

U.S. Patent No. 5,811,425 to Woods et al. refers to injectable depot forms of controlled release drugs made by forming microencapsule matrices of the drug in biodegradable polymers, liposomes, or microemulsions compatible with body tissues. U.S. Patent No. 5,811,422 to Lam et al. refers to controlled release compositions obtained by coupling a class of drugs to biodegradable polymers, such as polylactic acid, polyglycolic acid, copolymers of polylactic and polyglycolic acid, polyepsilon caprolactone, polyhydroxy butyric acid, etc.

U.S. Patent No. 5,811,404 to De Frees et al. refers to the use of liposomes having prolonged circulation half-lives to provide for the sustained release of drug compositions.

Nanoparticulate compositions addressed a need in the art for pharmaceutically-acceptable compositions containing poorly-water soluble agents. However, the known nanoparticulate compositions are not suitable for controlled-release formulations. There remains a need in the art for controlled release nanoparticulate compositions.

### SUMMARY OF THE INVENTION

This invention is directed to the surprising and unexpected discovery of new controlled release nanoparticulate compositions. The controlled release compositions provide for the therapeutically effective release of an incorporated drug or other substance in a patient for a time period ranging from about 2 to about 24 hours or longer.

The controlled release nanoparticulate compositions comprise a nanoparticulate drug or other agent to be administered, such as a crystalline or amorphous nanoparticulate drug or other agent, or a combination of a crystalline and amorphous nanoparticulate drug or other agent, having an effective average particle size, prior to inclusion in the composition, of less than about 1000 nm. The composition also comprises at least one surface stabilizer associated with the surface of the nanoparticulate drug or other agent. In addition, the controlled release nanoparticulate composition comprises one or more pharmaceutically acceptable rate-controlling polymers, which function to prolong release of the administered nanoparticulate drug or agent thereby resulting in controlled release. Optionally, one or more auxilary excipient materials can also be included in the controlled release composition.

Controlled release compositions according to this invention containing a nanoparticulate form of a poorly soluble drug are advantageous in that the improved bioavailability achieved by size reduction of the drug can be exploited to maintain an effective blood concentration over an extended period of time after administration.

Preferably, the effective average particle size of the nanoparticulate agent prior to inclusion in the controlled release nanoparticulate composition is less than about 1000 nm, less than about 800 nm, less than about 600 nm, less than about 400 nm, less than about 300 nm, less than about 250 nm, less than about 100 nm, or less than about 50 nm. Nanoparticulate compositions were first described in U.S. Patent No. 5,145,684 ("the '684 patent"), described above.

The present invention also provides dosage forms for the controlled release composition as described above in tablet form or in multiparticulate form to be administered in any conventional method, such as via oral, rectal, buccal, and vaginal routes. The tablet form may be, for instance, coated tablets, multilayer tablets, matrix tablets, and the like. The multiparticulate form may be, for instance, particles, pellets, mini-tablets, or the like.

In a first aspect of the invention, the nanoparticulate drug or other agent, at least one surface stabilizer, and one or more auxiliary excipient materials are compressed into tablet form prior to coating with a rate controlling polymer material.

In a second aspect, the nanoparticulate drug or other agent, at least one surface stabilizer, the rate controlling polymer material, and one or more auxiliary excipients are compressed together to form a controlled release matrix. The controlled release matrix may optionally be coated with a rate controlling polymer so as to provide additional controlled release properties.

In a third aspect, the nanoparticulate drug or other agent, at least one surface stabilizer, and one or more auxiliary excipient materials are compressed into the form of a multilayer tablet prior to coating with a rate controlling polymer material.

In a fourth aspect, the nanoparticulate drug or other agent and at least one surface stabilizer are dispersed in the rate controlling polymer material and compressed into the form of a multilayer tablet. The multilayer tablet may optionally be coated with a rate controlling polymer material so as to provide additional controlled release properties. In an alternative aspect, a first layer in such a multilayer tablet comprises a controlled release composition according to the invention and a second layer comprises a conventional active ingredient containing composition, such as an instant release composition.

In a fifth aspect, the nanoparticulate drug or other agent and at least one surface stabilizer are incorporated into a single layer or multilayer tablet containing osmagent surrounded by a semi-permeable membrane, with the semi-permeable membrane defining an orifice. In this embodiment the semi-permeable membrane is permeable to aqueous media, such as gastrointestinal fluids, but it is not permeable to the poorly soluble drug compound when in solution or when in other form. Such osmotic delivery systems are well known in the art, wherein infusion of fluid through the semi-permeable membrane causes the osmagent to swell thus driving the drug compound through the orifice defined by the semi-permeable membrane.

In a sixth aspect, the nanoparticulate drug or other agent, at least one surface stabilizer, one or more auxiliary excipients, and the rate controlling polymer material are combined into a multiparticulate form. The multiparticulate form preferably comprises discrete particles, pellets, mini-tablets, or combinations thereof. In a final oral dosage form the multiparticulate form may be encapsulated, for example in hard or soft gelatin capsules. Alternatively, a multiparticulate form may be incorporated into other final dosage forms such as a sachet. In the case of a multiparticulate form comprising discrete particles or pellets, the multiparticulate form may be compressed, optionally with additional auxiliary excipients, into the form of tablets. The compressed multiparticulate tablet may optionally be coated with rate controlling polymer material so as to provide additional controlled release properties.

The present invention further relates to processes for the manufacture of controlled release compositions in which a poorly soluble drug or other agent is present in nanoparticulate form. In one aspect, the method comprises: (1) forming a nanoparticulate composition comprising a poorly soluble drug or other agent to be administered and a surface stabilizer; (2) adding one or more pharmaceutically acceptable rate-controlling polymers, and (3) forming a solid dose form of the composition for administration. Pharmaceutically acceptable excipients can also be added to the composition for administration. Methods of making nanoparticulate compositions, which can comprise mechanical grinding, precipitation, or any other suitable size reduction process, are known in the art and are described in, for example, the '684 patent.

Yet another aspect of the present invention provides a method of treating a mammal, including a human, requiring extended administration of a drug or other agent with a controlled release nanoparticulate composition of the invention which releases an incorporated drug or other agent providing a desired effect for a period from about 2 to about 24 hours or longer. The controlled release nanoparticulate composition can be administered in any conventional method, such as via oral, rectal, buccal, and vaginal routes.

It is to be understood that both the foregoing general description and the following detailed description are exemplary and explanatory and are intended to provide further explanation of the invention as claimed. Other objects, advantages, and novel features will be readily apparent to those skilled in the art from the following detailed description of the invention.

### BRIEF DESCRIPTION OF THE FIGURES

- Figure 1:: Shows a graph of the cumulative % drug (naproxen) released over time using a nanoparticulate composition comprising 30% Klucel^{®} hydroxypropylcellulose (HPC) and 3% polyvinylpyrrolidone (PVP);
- Figure 2:: Shows a graph of the cumulative % drug (naproxen) released over time for three different nanoparticulate compositions having a hardness of 15, 25, and 35 kP;
- Figure 3:: Shows a graph of the cumulative % drug (naproxen) released over time for nanoparticulate compositions comprising different types of hydroxypropyl methylcellulose (HPMC);
- Figure 4:: Shows a graph of the cumulative % drug (naproxen) released over time for nanoparticulate compositions comprising one of six different types of HPMC;
- Figure 5:: Shows a graph of the cumulative % drug (naproxen) released over time for nanoparticulate compositions having varying amounts Lubritab^{®} (a hydrogenated vegetable oil);
- Figure 6:: Shows a graph comparing the cumulative % drug (naproxen) released over time for a spray-dried nanoparticulate formulation and a formulation of blended raw drug and stabilizer;
- Figure 7:: Shows a graph comparing the cumulative % drug (naproxen) released over time for nanoparticulate formulations comprising different concentrations of Methocel^{®} K100LV (HPMC);
- Figure 8:: Shows a graph comparing the cumulative % drug (naproxen) released over time for directly compressed and wet granulated nanoparticulate formulations of Klucel^{®} and Methocel^{®};
- Figure 9:: Shows the controlled release of nanoparticulate glipizide from directly compressed Methocel^{®} tablets;
- Figure 10:: Shows the mean *in vivo* plasma profiles of nifedipine after single dosed, fasted, administration in humans for (1) nifedipine containing controlled release matrix tablets coated with a controlled release coating according to the present invention as described in Example 12; and (2) a control composition;
- Figure 11:: Shows the mean *in vivo* plasma profiles of nifedipine after single dosed, fasted, administration in humans for (1) a nifedipine controlled release composition manufactured according to the present invention as described in Example 14; and (2) a control composition;
- Figure 12:: Shows the controlled release pattern of tablets containing 10% and 30% Carbopol^{®} 971P NF;
- Figure 13:: Shows a comparison of the controlled release pattern of tablets containing 5% Carbopol^{®} 971P NF and Carbopol^{®} 974P-NF; and
- Figure 14:: Shows the controlled release pattern of tablets containing 30% Eudragit^{®} grades RS PO and RL PO.

### DETAILED DESCRIPTION OF THE INVENTION

### A. CONTROLLED RELEASE NANOPARTICULATE COMPOSITIONS

This invention is directed to the surprising and unexpected discovery of new solid dose controlled release nanoparticulate compositions. It is expected that the controlled release compositions provide effective blood levels of an incorporated nanoparticulate drug or other agent in a patient for an extended period of time. Such a discovery was unexpected because the nanoparticulate size of the drug or other agent, resulting in a large surface area in relation to the volume, results in rapid dissolution of the drug or other agent following administration. Rapid dissolution is seemingly contrary to the goal of controlled release formulations.

As used herein, "controlled release" means the release of an agent such as a drug from a composition or dosage form in which the agent is released according to a desired profile over an extended period of time, such as from about 2 to about 24 hours or longer. Release over a longer time period is also contemplated as a "controlled release" dosage form of the present invention.

The solid dose controlled release nanoparticulate compositions of the invention comprise a crystalline or amorphous nanoparticulate drug or other agent to be administered, having an effective average particle size of less than about 1000 nm, at least one surface stabilizer associated with the surfaceof the drug or agent, and, additionally, one or more rate-controlling polymers. Preferably, the effective average particle size of the nanoparticulate drug is less than about 800 nm, less than about 600 nm, less than about 400 nm, less than about 300 nm, less than about 250 nm, less than about 100 nm, or less than about 50 nm. The crystalline form of a drug or other agent is distinguishable from a non-crystalline or amorphous phase of a drug or other agent.

### 1. Nanoparticulate Compositions

The starting nanoparticulate composition (prior to addition of the one or more rate-controlling polymers) comprises a drug or other agent to be administered and at least one surface stabilizer associated with the surface of the nanoparticulate drug or agent.

### a. Agent to be Administered

The nanoparticles of the invention comprise a therapeutic agent, diagnostic agent, or other agent to be administered for controlled release. A therapeutic agent can be a drug or pharmaceutical, and a diagnostic agent is typically a contrast agent, such as an x-ray contrast agent, or any other type of diagnostic material. The drug or diagnostic agent exists as a discrete, crystalline phase, as an amorphous phase, or as a combination thereof. The crystalline phase differs from a non-crystalline or amorphous phase that results from precipitation techniques, such as those described in EPO 275,796.

The invention can be practiced with a wide variety of drugs or diagnostic agents. The drug or diagnostic agent is preferably present in an essentially pure form, is poorly water soluble, and is dispersible in at least one liquid medium. By "poorly water soluble" it is meant that the drug or diagnostic agent has a solubility in the liquid dispersion medium of less than about 30 mg/ml, preferably less than about 10 mg/ml, and preferably less than about 1 mg/ml.

Suitable drugs or diagnostic agents include those intended for controlled release delivery. Preferable drug classes include those that have short half-lives for clearance.

The drug can be selected from a variety of known classes of drugs, including, for example, analgesics, anti-inflammatory agents, anthelmintics, anti-arrhythmic agents, antiasthma agents, antibiotics (including penicillins), anticoagulants, antidepressants, antidiabetic agents, antiepileptics, antihistamines, antitussives, antihypertensive agents, antimuscarinic agents, antimycobacterial agents, antineoplastic agents, antipyretics, immunosuppressants, immunostimulants, antithyroid agents, antiviral agents, anxiolytic sedatives (hypnotics and neuroleptics), astringents, beta-adrenoceptor blocking agents, blood products and substitutes, bronchodilators, cardiac inotropic agents, chemotherapeutics, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, proteins, polypeptides, parasympathomimetics, parathyroid calcitonin and biphosphonates, prostaglandins, radio-pharmaceuticals, hormones, sex hormones (including steroids), anti-allergic agents, stimulants and anoretics, sympathomimetics, thyroid agents, vaccines, vasodilators, and xanthines.

A description of these classes of drugs and diagnostic agents and a listing of species within each class can be found, for instance, in Martindale, The Extra Pharmacopoeia, Twenty-ninth Edition (The Pharmaceutical Press, London, 1989), specifically incorporated by reference. The drugs or diagnostic agents are commercially available and/or can be prepared by techniques known in the art.

Poorly water soluble drugs which may be suitably used in the practice of the present invention include but are not limited to alprazolam, amiodarone, amlodipine, astemizole, atenolol, azathioprine, azelatine, beclomethasone, budesonide, buprenorphine, butalbital, carbamazepine, carbidopa, cefotaxime, cephalexin, cholestyramine, ciprofloxacin, cisapride, cisplatin, clarithromycin, clonazepam, clozapine, cyclosporin, diazepam, diclofenac sodium, digoxin, dipyridamole, divalproex, dobutamine, doxazosin, enalapril, estradiol, etodolac, etoposide, famotidine, felodipine, fentanyl citrate, fexofenadine, finasteride, fluconazole, flunisolide, flurbiprofen, fluvoxamine, furosemide, glipizide, gliburide, ibuprofen, isosorbide dinitrate, isotretinoin, isradipine, itraconazole, ketoconazole, ketoprofen, lamotrigine, lansoprazole, loperamide, loratadine, lorazepam, lovastatin, medroxyprogesterone, mefenamic acid, methylprednisolone, midazolam, mometasone, nabumetone, naproxen, nicergoline, nifedipine, norfloxacin, omeprazole, paclitaxel, phenytoin, piroxicam, quinapril, ramipril, risperidone, sertraline, simvastatin, terbinafine, terfenadine, triamcinolone, valproic acid, zolpidem, or pharmaceutically acceptable salts of any of the abovementioned drugs.

### b. Surface Stabilizers

Useful surface stabilizers, which are known in the art and described, for example, in the '684 patent, are believed to include those which physically adhere to the surface of the drug or agent but do not chemically bond to or interact with the drug or agent. The surface stabilizer is associated with the surface of the drug or agent in an amount sufficient to maintain an effective average particle size of less than about 1000 nm. Furthermore, the individual molecules of the surface stabilizer are essentially free of intermolecular cross-linkages.

Suitable surface stabilizers can preferably be selected from known organic and inorganic pharmaceutical excipients. Such excipients include various polymers, low molecular weight oligomers, natural products, and surfactants. Preferred surface stabilizers include nonionic and ionic surfactants.

Representative examples of surface stabilizers include gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g.*, macrogol ethers such as cetomacrogol 1000), polyoxyethylene castor oil derivatives, polyoxyethylene sorbitan fatty acid esters (*e.g.*, the commercially available Tweens^{®} such as *e.g.*, Tween 20^{®} and Tween 80^{®} (ICI Specialty Chemicals)); polyethylene glycols (*e.g.*, Carbowaxs 3550^{®} and 934^{®} (Union Carbide)), polyoxyethylene stearates, colloidal silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethyl-cellulose phthalate, noncrystalline cellulose, magnesium aluminum silicate, triethanolamine, polyvinyl alcohol (PVA), polyvinylpyrrolidone (PVP), 4-(1,1,3,3-tetramethylbutyl)-phenol polymer with ethylene oxide and formaldehyde (also known as tyloxapol, superione, and triton), poloxamers (*e.g.*, Pluronics F68^{®} and F108^{®}, which are block copolymers of ethylene oxide and propylene oxide); poloxamines (*e.g.*, Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®}(T-1508) (BASF Wyandotte Corporation), dialkylesters of sodium sulfosuccinic acid (*e.g*., Aerosol OT^{®}, which is a dioctyl ester of sodium sulfosuccinic acid (American Cyanamid)); Duponol P^{®}, which is a sodium lauryl sulfate (DuPont); Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-lOG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂0H)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; and the like.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 1986), specifically incorporated by reference.

### c. Particle Size

By "an effective average particle size of less than about 1000 nm" it is meant that at least 50% of the drug/agent particles have an average particle size of less than about 1000 nm when measured by light scattering techniques. Preferably, at least 70% of the particles have an average particle size of less than the effective average, i.e., about 1000 nm, more preferably at least about 90% of the particles have an average particle size of less than the effective average.

As used herein, particle size is determined on the basis of the weight average particle size as measured by conventional particle size measuring techniques well known to those skilled in the art. Such techniques include, for example, sedimentation field flow fractionation, photon correlation spectroscopy, light scattering, and disk centrifugation. By "an effective average particle size of less than about 1000 nm" it is meant that at least 70% of the particles, by weight, have a particle size of less than about 1000 nm when measured by the above-noted techniques. In preferred embodiments, the effective average particle size is less than about 800 nm, less than about 600 nm, less than about 400 nm, less than about 300 nm, less than about 250 nm, less than about 100 nm, or less than about 50 nm.

As used herein, the mean diameter of 50% of the particles, D_{v,50}, refers to the volume average diameter of 50% of the particles or the value below which 50% of the particles have an equivalent volume diameter.

### 2. Rate-controlling Polymers

The present invention identifies pharmaceutically acceptable rate-controlling polymers (also referred to herein as rate controlling polymer material) that unexpectedly provide excellent controlled release properties for nanoparticulate compositions. Rate-controlling polymers include hydrophilic polymers, hydrophobic polymers, and mixtures of hydrophobic and hydrophilic polymers that are capable of retarding the release of a drug compound from a composition or dosage form of the present invention.

Particularly useful rate-controlling polymers for causing an effective controlled release of administered drug or agent following administration include plant exudates (gum arabic), seaweed extracts (agar), plant seed gums or mucilages (guar gum), cereal gums (starches), fermentation gums (dextran), animal products (gelatin), hydroxyalkyl celluloses such as hydroxypropyl cellulose (HPC), hydroxyethyl cellulose (HEC), hydroxypropyl methylcelluose (HPMC), and sodium carboxymethylcellulose (CMC), guar, pectin, and carrageenan. Additional polymers include poly(ethylene) oxide, alkyl cellulose such as ethyl cellulose and methyl cellulose, carboxymethyl cellulose, hydrophilic cellulose derivatives, polyethylene glycol, polyvinylpyrrolidone, cellulose acetate, cellulose acetate butyrate, cellulose acetate phthalate, cellulose acetate trimellitate, polyvinyl acetate phthalate, hydroxypropylmethyl cellulose phthalate, hydroxypropylmethyl cellulose acetate succinate, polyvinyl acetaldiethylamino acetate, poly(alkylmethacrylate) and poly(vinyl acetate). Other suitable hydrophobic polymers include polymers and/or copolymers derived from acrylic or methacrylic acid and their respective esters, waxes, shellac, and hydrogenated vegetable oils. Two or more rate-controlling polymers can be used in combination. The polymers are commercially available and/or can be prepared by techniques known in the art.

### 3. Other Pharmaceutical Excipients

Pharmaceutical compositions according to the invention may also comprise one or more auxiliary excipients such as binding agents, diluents, lubricating agents, plasticisers, anti-tack agent, opacifying agents, suspending agents, sweeteners, flavoring agents, preservatives, buffers, wetting agents, disintegrants, pigments, and other excipients. Such excipients are known in the art. As will be appreciated by those skilled in the art, the exact choice of excipients and their relative amounts will depend to some extent on the dosage form into which the controlled release composition is incorporated.

Suitable diluents include for example pharmaceutically acceptable inert fillers such as microcrystalline cellulose, lactose, dibasic calcium phosphate, saccharides, and/or mixtures of any of the foregoing. Examples of diluents include microcrystalline cellulose such as Avicel pH101, Avicel pH102, and Avicel pH112; lactose such as lactose monohydrate, lactose anhydrous, and Pharmatose DCL21; dibasic calcium phosphate such as Emcompress; mannitol; starch; sorbitol; sucrose; and glucose. The diluent, if present, is preferably used in an amount of from about 5 mg to about 800 mg per dosage unit, more preferably from about 10 mg to about 600 mg per dosage unit and most preferably from about 20 mg to about 400 mg per dosage unit.

Examples of binding agents are various celluloses and cross-linked polyvinylpyrrolidone.

Suitable lubricants, including agents that act on the flowability of the powder to be compressed, are colloidal silicon dioxide, such as Aerosil 200; talc, stearic acid, magnesium stearate, calcium stearate, stearic acid, and silica gel.

Examples of sweeteners are any natural or artificial sweetener, such as sucrose, xylitol, sodium saccharin, cyclamate, aspartame, and acsulfame. Examples of flavoring agents are Magnasweet (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

### 4. Quantities of Nanoparticulate Composition and Rate-controlling Polymer(s)

The relative amount of nanoparticulate agent in the controlled release compositions of the invention can vary widely and can depend upon, for example, the agent selected for controlled release delivery. The poorly soluble drug or pharmaceutically acceptable salt thereof may be present in any amount which is sufficient to elicit a therapeutic effect and, where applicable, may be present either substantially in the form of one optically pure enantiomer or as a mixture, racemic or otherwise, of enantiomers. The amount of poorly soluble drug compound, or pharmaceutically acceptable salt thereof, in the controlled release composition of the present invention is suitably in the range of from about 1 µg to about 800 mg, preferably in the range of from about 0.25 mg to about 600 mg and more preferably in the range of from about 1 mg to about 500 mg.

The nanoparticulate agent, preferably in combination with the surface stabilizer, can be present in the controlled release compositions of the invention in an amount of about 95% to about 5%, preferably about 80% to about 10% by weight based on the total weight of the dry composition.

The one or more rate-controlling polymers can be present in an amount of about 5% to about 95%, preferably about 10% to about 65% by weight based on the total weight of the dry composition.

### 5. Optimization of Other Variables for Increasing Controlled Release

Other than selection of the one or more rate-controlling polymers, hardness of the tablet is the factor which contributes most to extended controlled release of the administered agent. A hardness of about 10 kP to about 50 kP is preferred, with a hardness of about 30 to about 35 kP being most preferred. Factors such as wet-granulation of the rate-controlling polymer and an increase in the concentration of the rate-controlling polymer allow for a more controlled release, while factors such as micronization of the rate-controlling polymer give a more immediate release of the administered agent.

### B. METHODS OF MAKING CONTROLLED RELEASE NANOPARTICULATE DOSAGE FORMS

In another aspect of the invention there is provided a method of preparing controlled release nanoparticulate formulations. The method comprises: (1) forming a nanoparticulate composition comprising an agent to be administered and, preferably, a surface stabilizer; (2) adding one or more rate-controlling polymers, and (3) forming a solid dose form of the composition for administration. Pharmaceutically acceptable excipients can also be added to the composition for administration. Methods of making nanoparticulate compositions, which can comprise mechanical grinding, precipitation, or any other suitable size reduction process, are known in the art and are described in, for example, the '684 patent. A redispersing agent or combination of redispersing agents may be included to facilitate processing of the nanoparticulate drug.

Methods for making solid dose pharmaceutical formulations are known in the art, and such methods can be employed in the present invention. Exemplary solid dose controlled release formulations of the invention can be prepared by, for example, combining the one or more rate-controlling polymers with a raw nanoparticulate mixture obtained after size reduction of an agent to be administered. The resultant composition can be formulated into tablets for oral administration. Alternatively, the one or more rate-controlling polymers can be combined with a nanoparticulate dispersion that has been spray dried.

Oral dosage forms of the controlled release composition according to the present invention can be in the form of tablets or can be multiparticulate. The term "tablet" or "tablets" as used herein includes, but is not limited to, instant release (IR) tablets, matrix tablets, multilayer tablets, and multilayer matrix tablets which may be optionally coated with one or more coating materials. The term "tablet" also includes osmotic delivery systems in which a drug compound is combined with an osmagent (and optionally other excipients) and coated with a semi-permeable membrane, the semi-permeable membrane defining an orifice through which the drug compound may be released. Tablet oral dosage forms particularly useful in the practice of the invention include those selected from the group consisting of coated IR tablets, matrix tablets, coated matrix tablets, multilayer tablets, coated multilayer tablets, multilayer matrix tablets, and coated multilayer matrix tablets. The term "multiparticulate" as used herein includes discrete particles, pellets, mini-tablets, and mixtures or combinations thereof. If the oral form is a multiparticulate capsule, such hard or soft gelatin capsules can suitably be used to contain the multiparticulate. A multiparticulate oral dosage form according to the invention may comprise a blend of two or more populations of particles, pellets, or mini-tablets having different *in vitro* and/or *in vivo* release characteristics. For example, a multiparticulate oral dosage form may comprise a blend of an instant release component and a delayed release component contained in a suitable capsule.

If desired, the multiparticulate may be coated with a layer containing controlled release polymer material. Alternatively, the multiparticulate and one or more auxiliary excipient materials can be compressed into tablet form such as a multilayer tablet. Typically, a multilayer tablet may comprise two layers containing the same or different levels of the same active ingredient having the same or different release characteristics. Alternatively, a multilayer tablet may contain different active ingredient in each layer. Multilayer tablets may optionally be coated with a controlled release polymer so as to provide additional controlled release properties.

In one embodiment of the invention the rate controlling polymer material is applied as a coating to tablets comprising the poorly soluble drug compound and any auxiliary excipients which may be required. The coating may be applied to the tablets by any suitable technique. Such techniques will be apparent to those skilled in the art. Particularly useful for application of the coating is the technique of spray coating, carried out for instance using a fluidised bed coating apparatus or using a side vented coating pan. Suitable auxiliary excipients and/or additives may be added to the coating formulation. For example, it may be desirable to add plasticisers, glidants, anti-tack agents, pigments, and other excipients to the coating formulation. The coating may be applied to the tablets in any amount which is sufficient to give the desired degree of controlled release.

In one embodiment a process for the manufacture of a controlled release composition comprises the steps of: (i) spray drying a nanoparticulate dispersion of a poorly soluble drug, optionally in the presence of a surfactant or a surface stabilizer, to form a redispersible material; (ii) blending the redispersible material with auxiliary excipients to form a blend, (iii) compressing the blend into tablets, and (iv) coating the tablets with a rate controlling polymer material.

In an another embodiment, a process for the manufacture of a controlled release composition comprises the steps of: (i) spray drying a nanoparticulate dispersion of a poorly soluble drug, optionally in the presence of a surfactant or a surface stabilizer, to form a redispersible material; (ii) blending the redispersible material with a rate controlling polymer material and optionally auxiliary excipients to form a blend, and (iii) compressing the blend to form tablets. The process may optionally comprise the additional step of coating the tablets with an additional rate controlling polymer material.

### 1. Spray Drying of Nanoparticulate Dispersions

Solid dose forms of nanoparticulate dispersions can be prepared by drying the nanoparticulate formulation following size reduction. A preferred drying method is spray drying. The spray drying process is used to obtain a nanoparticulate powder following the size reduction process used to transform the drug into nanoparticulate sized particles. Such a nanoparticulate powder can be formulated into tablets for oral administration.

In an exemplary spray drying process, the nanoparticulate drug suspension is fed to an atomizer using a peristaltic pump and atomized into a fine spray of droplets. The spray is contacted with hot air in the drying chamber resulting in the evaporation of moisture from the droplets. The resulting spray is passed into a cyclone where the powder is separated and collected. The spray dryer can be assembled in a co-current configuration with a rotary atomization nozzle and the nanosuspension can be fed to the rotary atomizer using a peristaltic pump.

### 2. Tableting

The controlled release nanoparticulate formulations of the invention can be in the form of tablets for oral administration. Preparation of such tablets can be by pharmaceutical compression or molding techniques known in the art. The tablets of the invention may take any appropriate shape, such as discoid, round, oval, oblong, cylindrical, triangular, hexagonal, and the like.

The tablets may be coated or uncoated. If coated they may be sugar-coated (to cover objectionable tastes or odors and to protect against oxidation), film coated (a thin film of water soluble matter for similar purposes), or enteric coated (to resist dissolution in gastric fluid but allow disintegration of the coating in the small intestine).

Tableting techniques known to one of ordinary skill in the art are described in, for example, the 18th edition of Remington's Pharmaceutical Sciences, Chapter 89, pp. 1633-1658 (Mach Publishing Company, 1990), which is specifically incorporated by reference. In the simplest procedure, the ingredients (except for any lubricant) are blended together to provide a mixture having the active ingredient uniformly dispersed throughout. A lubricant can then be added and blended, and the tablets are compressed using an appropriate tableting machine.

Formulations suitable for tableting are prepared using, for example, a V-blender (Blend Master Lab Blender, Patterson Kelley Co.). In an exemplary method, the nanoparticulate composition and the one or more rate-controlling polymers are added to the V-blender and blended periodically, followed by the addition of other excipients, such as lactose, magnesium stearate, or PVP, followed by periodic blending in the V-Blender.

Tableting can be accomplished by using, for example, a Carver Press (Carver Laboratory Equipment). In such a method, the correct amount of material is loaded into the punches, followed by pressing together at the appropriate pressure and time interval, and removal of the formed tablet.

Yet another exemplary method for creating tablets is wet-granulation. Wet-granulation comprises mixing water and/or granulating fluid to the dry materials (nanoparticulate composition (comprising a drug and surface stabilizer), rate-controlling polymer, and any additives). After thorough granulation, the material is sieved through a coarse mesh screen and dried. The material is then re-sieved through a fine mesh screen and blended with, for example, magnesium stearate, followed by tableting to create tablets.

Tablets are tested to determine that they meet the correct hardness specifications. An exemplary tablet hardness tester is an Erweka TBH 30 (Erweka Instruments, Inc.).

### C. ADMINISTRATION OF CONTROLLED RELEASE NANOPARTICULATE COMPOSITIONS OR DOSAGE FORMS

Yet another aspect of the present invention provides a method of treating a mammal, including a human, requiring extended administration of a drug or other agent. The administered controlled release nanoparticulate composition releases an incorporated drug or other agent over a prolonged period of time providing a desired effect for a period from about 2 to about 24 hours or more.

In general, the compositions of the invention will be administered to a mammalian subject in need thereof using a level of drug or agent that is sufficient to provide the desired physiological effect via any conventional method, such as orally, rectally, buccally, or via the vagina. The mammalian subject may be a domestic animal or pet but preferably is a human subject. The level of drug or agent needed to give the desired physiological result is readily determined by one of ordinary skill in the art by referring to standard texts, such as *Goodman and Gillman* and the *Physician's Desk Reference.*

The following examples are given to illustrate the present invention. It should be understood, however, that the invention is not to be limited to the specific conditions or details described in these examples. Throughout the specification, any and all references to a publicly available documents are specifically incorporated into this patent application by reference.

### Example 1

The purpose of this experiment was to demonstrate a reasonable amount of controlled release with a nanoparticulate drug formulation.

29% w/w spray-dried nanoparticulate naproxen intermediate (SDI) (containing 93% w/w nanoparticulate naproxen and 7% w/w polyvinylpyrrolidone (PVP) as a surface stabilizer (sieve #20)), 30% w/w Klucel^{®} HPC polymer (sieve #40), 40% w/w lactose (Foremost #316 Fast-fib, sieve #40), and 1% w/w magnesium stearate (Spectrum, sieve #40) were combined as follows to form a controlled release nanoparticulate formulation tablet to be tested.

The average effective particle size of the nanoparticulate naproxen prior to spray-drying to form spray-dried nanoparticulate naproxen intermediate was 226 nm, with 90% of the particles having a size of less than 297 nm. The spray-dried powder had a mean particle size of about 26 µm. This particle size information for the naproxen SDI is applicable to the following Examples 2-10.

(The sources given in this example for the naproxen SDI, PVP, Klucel^{®} (an HPC polymer), lactose, and magnesium stearate are also applicable to the following examples.)

The naproxen SDI and Klucel^{®} were added to a V-blender (Blend Master Lab Blender, Patterson Kelley Co.) and blended for 10 min. The lactose was then added to the blender and blended for 10 min. Finally, the magnesium stearate was added to the blender and blended for 3 min.

This material was formed into tablets using a Carver Press (Carver Laboratory equipment, model #3912). The resultant tablets had a weight of 500 mg and a hardness of about 9 to about 12 kP

### Testing for Controlled Release

A Distek Dissolution System (used with the Hewlett Packard Diode Array Spectrophotometer 8452A and the Hewlett Packard Flow Control device model 89092A) was used in testing for controlled release. The temperature (37°C) and agitation of this instrument simulates the body system as it attempts to dissolve the drug in the tablet.

A phosphate buffer at pH 7.4 is used for the testing medium, prepared as follows: 230.0 grams of sodium phosphate dibasic, anhydrous (J.T. Baker) plus 52.4 grams of sodium phosphate monobasic, dihydrate (J.T. Baker) added to 20.0 liters of deionized water and stirred at 2300 rpm for two hours.

Phosphate buffer (900 ml) and a tablet were placed into a container of the Distek System at 37°C. The tablets were agitated, resulting in dissolution of the tablets within a range of 40-50 min. Such a time period is not suitable for controlled release applications.

### Example 2

The purpose of this experiment was to demonstrate controlled release with a nanoparticulate drug formulation.

To improve the controlled release characteristics of the formed tablets, (i) the weight of the tablet was increased from 500 to 750 mg, (ii) the hardness of the tablet was increased from 9-12 to 35-37 kP; and (iii) 3% extra PVP was added as a binder agent in place of 3% lactose.

Naproxen SDI (containing 93% w/w nanoparticulate naproxen and 7% w/w PVP), 30% w/w Klucel^{®} and 3% w/w PVP (Plasdone K-90 (Povidone USP), ISP Technologies) were combined as in Example 1 to form a tablet of 750 mg with a hardness of 35-37 kP (the PVP was added after addition of the lactose and blended for 5 additional min. in the V-Blender prior to tableting). Quantities of each component in the tablet are given below (mg).

| **Naproxen SDI** | **Klucel^{®}** | **Lactose** | **PVP** | **Mg Stearate** |
|---|---|---|---|---|
| 217.5 | 225 | 277.5 | 22.5 | 7.5 |

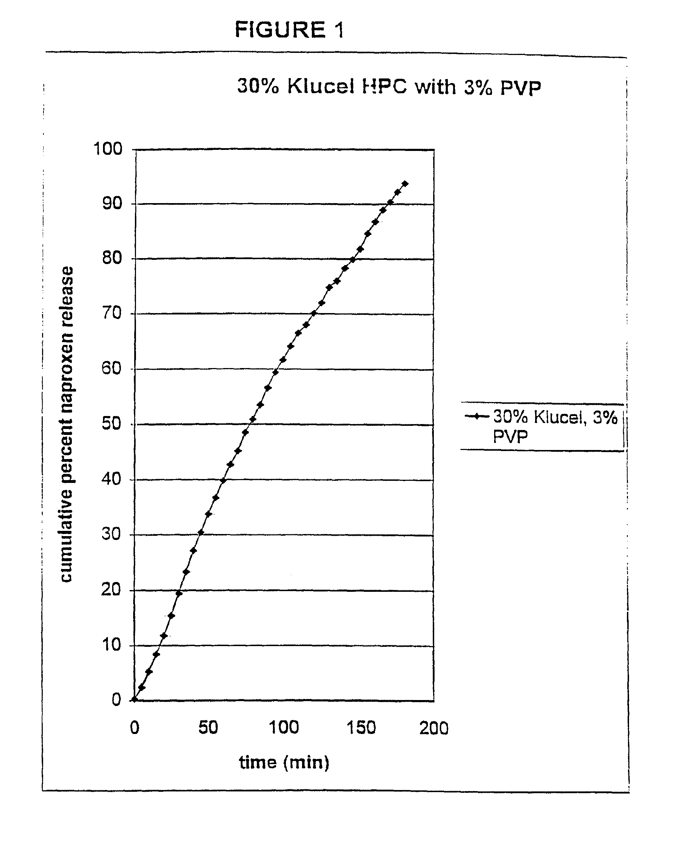

Following testing with the Distek Dissolution System, the results demonstrated a steady controlled release of drug over a three hour time period, as shown in Figure 1.

### Example 3

The purpose of this experiment was to determine the effects of the hardness of a tablet on controlled release of the nanoparticulate agent.

Three separate hardnesses were tested simultaneously: 15 kP, 25 kP, and 35 kP. Tablets were made as in Example 1, comprising 29% naproxen SDI, 30% Klucel^{®}, and 3% PVP. Quantities of each component in each of the tablet formulations are given below (mg).

| **Hardness** | **Naproxen SDI** | **Klucel^{®}** | **Lactose** | **PVP** | **Mg Stearate** |
|---|---|---|---|---|---|
| 15 | 217.5 | 225 | 277.5 | 22.5 | 7.5 |
| 25 | 217.5 | 225 | 277.5 | 22.5 | 7.5 |
| 35 | 217.5 | 225 | 277.5 | 22.5 | 7.5 |

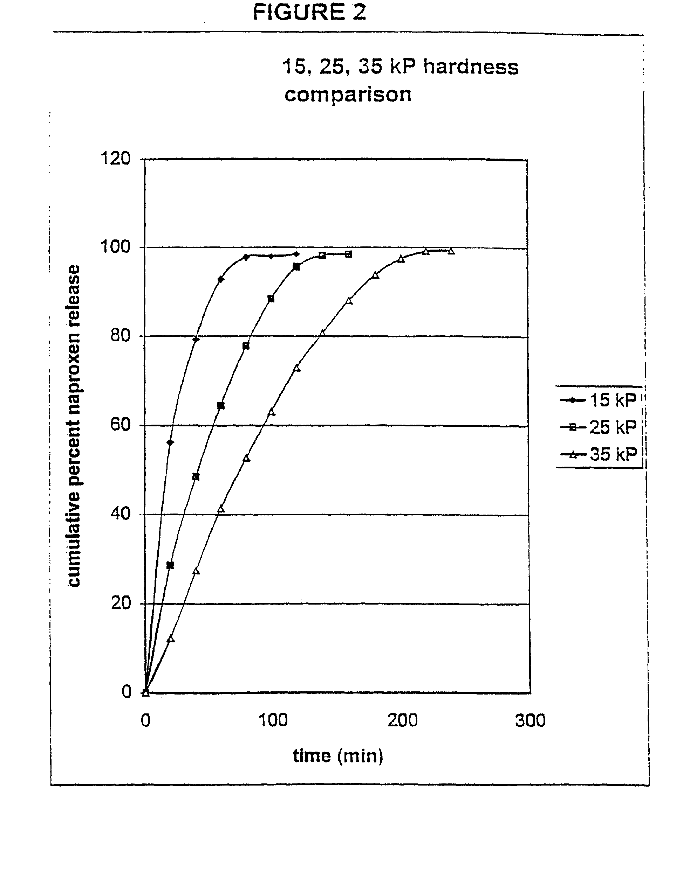

The results shown in Figure 2 demonstrate that as the hardness of a tablet increases, the controlled release characteristics of the tablet also steadily increase. Tablets having a hardness of about 15 kP, 25 kP, and 35 kP released naproxen for about 65 min., 140 min., and 240 min., respectively, showing a direct correlation between tablet hardness and increased controlled release of the administered agent.

### Example 4

The purpose of this experiment was to compare the controlled release characteristics of two different rate-controlling polymers: KIucel^{®} HPC and Shinetzu^{®} L-HPC.

Tablets were made as in Example 1, with 20% KIucel^{®} HPC (without the 3% PVP K-90) and with 20% Shinetzu^{®} L-HPC. Quantities of each component in each of the tablet formulations are given below (mg).

| **Naproxen SDI** | **Klucel^{®} HPC** | **Shinetzu^{®} L-HPC** | **Lactose** | **Mg Stearate** |
|---|---|---|---|---|
| 292.5 | 150 | 0 | 300 | 7.5 |
| 292.5 | 0 | 150 | 300 | 7.5 |

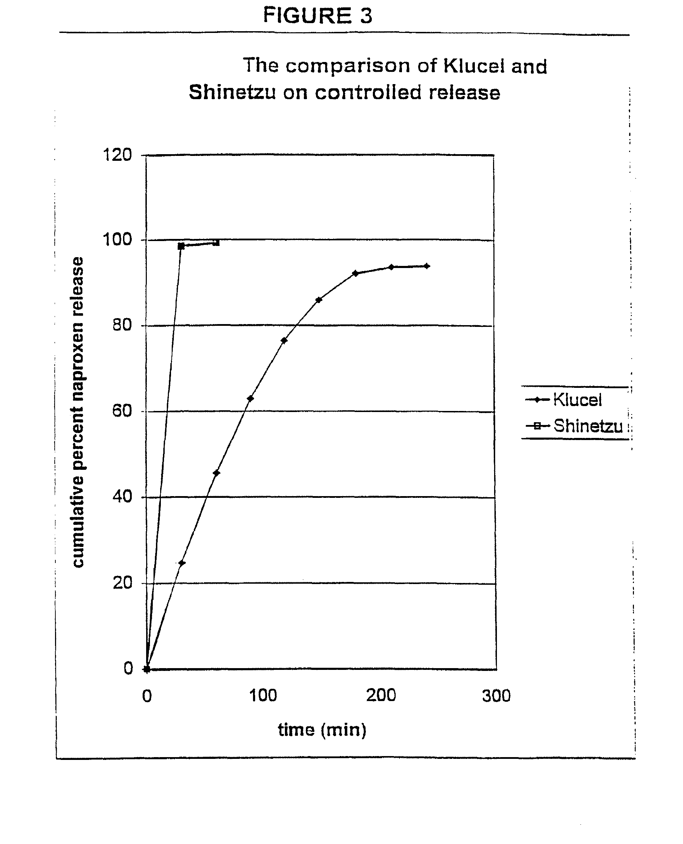

The resultant tablets had a hardness of 35 kP. The results, shown in Figure 3, demonstrate that the tablet with 20% KIucel^{®} as the polymer completely released within three to four hours, and the tablet with 20% Shinetzu^{®} L- HPC as the polymer allowed the tablet to dissolve in only one hour.

### Example 5

The purpose of this experiment was to compare the controlled release characteristics of different grades of Methocel^{®} hydroxypropyl methyl cellulose (HPMC) used as the rate-controlling polymer: (i) Methocel^{®} K4M, (ii) Methocel^{®} E4M, (iii) Methocel^{®} K15M, (iv) Methocel^{®} K100LV, (v) Methocel^{®} K100LV, and (vi) Methocel^{®} E10M.

Tablets were prepared as in Example 1, using a 20% concentration of Methocel^{®} HPMC. Quantities of each component in each of the tablet formulations are given below (mg).

| **Naproxen SDI** | **Methocel^{®} HPMC** | **Lactose** | **Mg Stearate** |
|---|---|---|---|
| 292.5 | 150 (K4M) | 300 | 7.5 |
| 292.5 | 150 (E4M) | 300 | 7.5 |
| 292.5 | 150(K15M) | 300 | 7.5 |
| 292.5 | 150(K100LV) | 300 | 7.5 |
| 292.5 | 150(K100LV) | 300 | 7.5 |
| 292.5 | 150HPMCE10M | 300 | 7.5 |

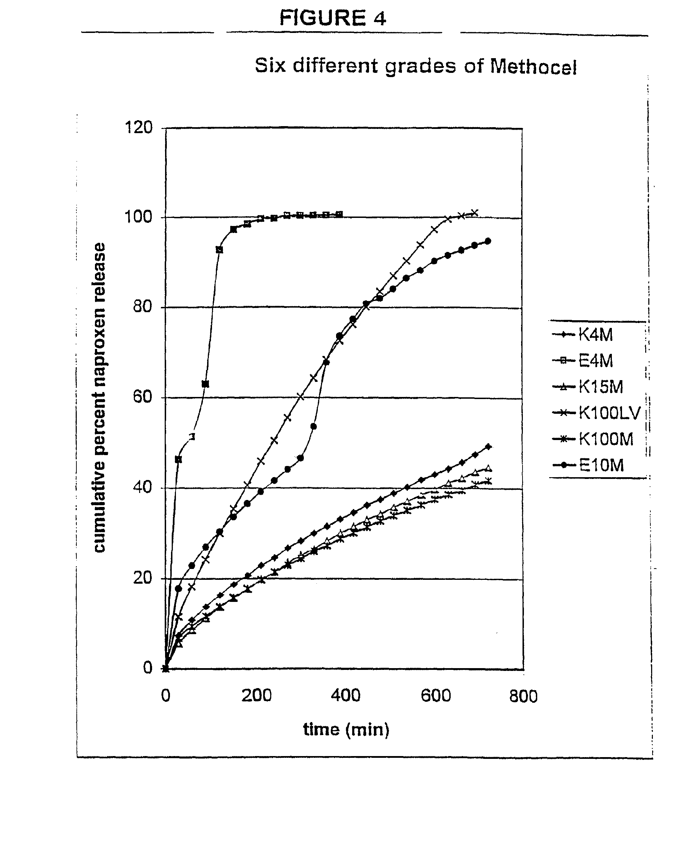

The tablets had a hardness of about 35 to about 37 kP. Each of the Methocel^{®} grades tested in the Distek Dissolution system, was found to exert some extent of controlled release on the nanoparticulate formulation, as shown in Figure 4. Methocel^{®} grades K4M, K15M, and K100M gave an extreme amount of controlled release (40-50% in 12 hours), Methocel^{®} grade E4M dissolved in only about three hours, and Methocel^{®} grades K100LV and E10M gave a release over about 12 to about 14 hours.

### Example 6

The purpose of this example was to determine the effect of adding hydrogenated vegetable oil (Lubritab^{®}) to controlled release of a nanoparticulate agent.

Tablets were prepared as in Example 1, with 30% KIucel^{®} used as the rate-controlling polymer. 3% Lubritab^{®} (Mendel, a Penwest Company) was used in the tablets. The tablets had a hardness of 20-22 kP. Quantities of each component in each of the tablet formulations are given below (mg).

| **Naproxen SDI** | **Klucel^{®}** | **Lactose** | **Lubritab^{®}** | **Mg. Stearate** |
|---|---|---|---|---|
| 217.5 | 225 | 300 | 0 | 7.5 |
| 217.5 | 225 | 262.5 | 37.5 | 7.5 |
| 217.5 | 225 | 225 | 75 | 7.5 |
| 217.5 | 225 | 150 | 150 | 7.5 |

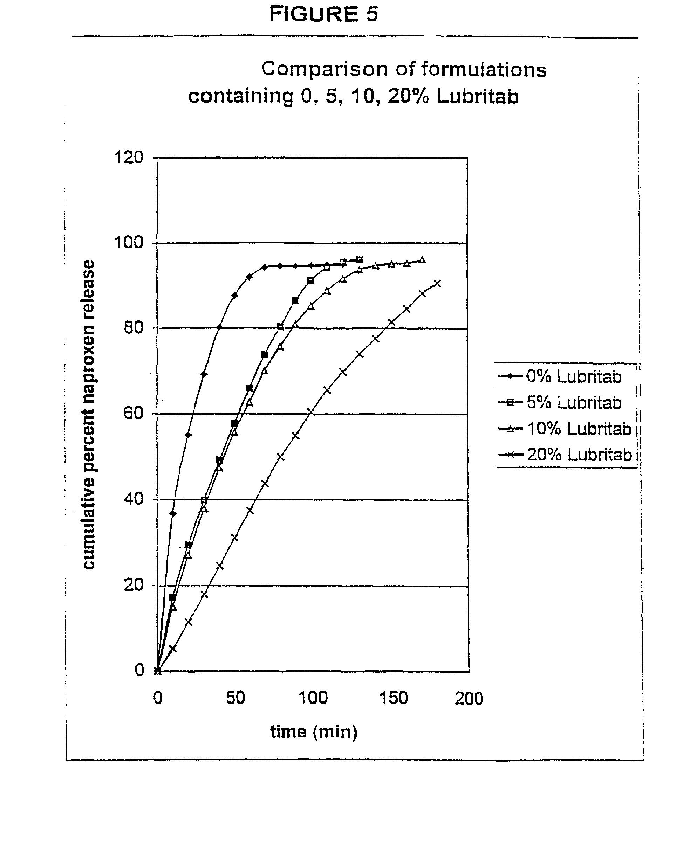

As shown in Figure 5, the addition of Lubritab^{®} to a nanoparticulate formulation can allow for an increase in controlled release of the administered agent. While the composition containing 0% Lubritab^{®} was completely released at about 60 min., the composition containing 20% Lubritab^{®} was released over about 175 min.

### Example 7

The purpose of this example was to compare the controlled release properties of a composition of a spray-dried nanoparticulate formulation mixed with a rate-controlling polymer and a powder composition of unmilled naproxen and surface stabilizer blended with a rate-controlling polymer.

Tablets were prepared as in Example 1. The concentration of the administered agent (naproxen) and surface stabilizer, PVP, was the same for both compositions: 93% naproxen and 7% PVP. The rate-controlling polymer used was Methocel^{®} K100LV in a concentration of 20%. Quantities of each component in each of the tablet formulations are given below (mg).

| **Naproxen SDI** | **Naproxen + PVP** | **Methocel^{®} K100LV** | **Lactose** | **Mg Stearate** |
|---|---|---|---|---|
| 292.5 | 0 | 150 | 300 | 7.5 |
| 0 | 292.5 | 150 | 300 | 7.5 |

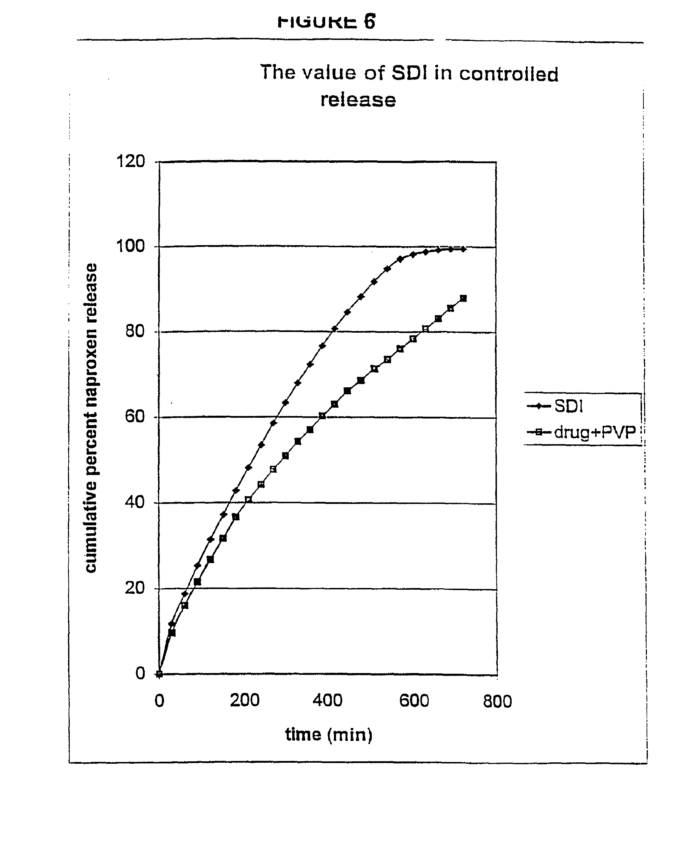

The tablets had a hardness of 30 kP. As shown in Figure 6, the composition of raw drug and surface stabilizer blended with a rate-controlling polymer had a more prolonged release as compared to the composition of the spray-dried nanoparticulate formulation mixed with a rate-controlling polymer. The results indicate that complete release of the composition of raw drug and stabilizer blended with a rate-controlling polymer occurred after about 10 hours, while complete release of the spray-dried nanoparticulate formulation mixed with a rate-controlling polymer was expected to occur after about 13 to about 14 hours (complete release of the latter composition had not occurred after 12 hours, when the results were analyzed).

### Example 8

The purpose of this example was to determine the effect of rate-controlling polymer concentration on the controlled release characteristics of nanoparticulate formulations.

The first test determined the controlled release characteristics of a nanoparticulate formulation comprising 5% Methocel^{®} K100LV, and the second test determined the controlled release characteristics of a nanoparticulate formulation comprising 10% Methocel^{®} K100LV. Controlled release characteristics of a nanoparticulate formulation comprising 20% Methocel^{®} K100LV were obtained in Example 9 (Figure 6) and are repeated here.

Tablets were prepared as in Example 1, with quantities of each component in each of the tablet formulations are given below (mg).

| **Naproxen SDI** | **Methocel^{®} K100LV** | **Lactose** | **Mg Stearate** |
|---|---|---|---|
| 405 | 37.5 | 300 | 7.5 |
| 367.5 | 75 | 300 | 7.5 |
| 292.5 | 150 | 300 | 7.5 |

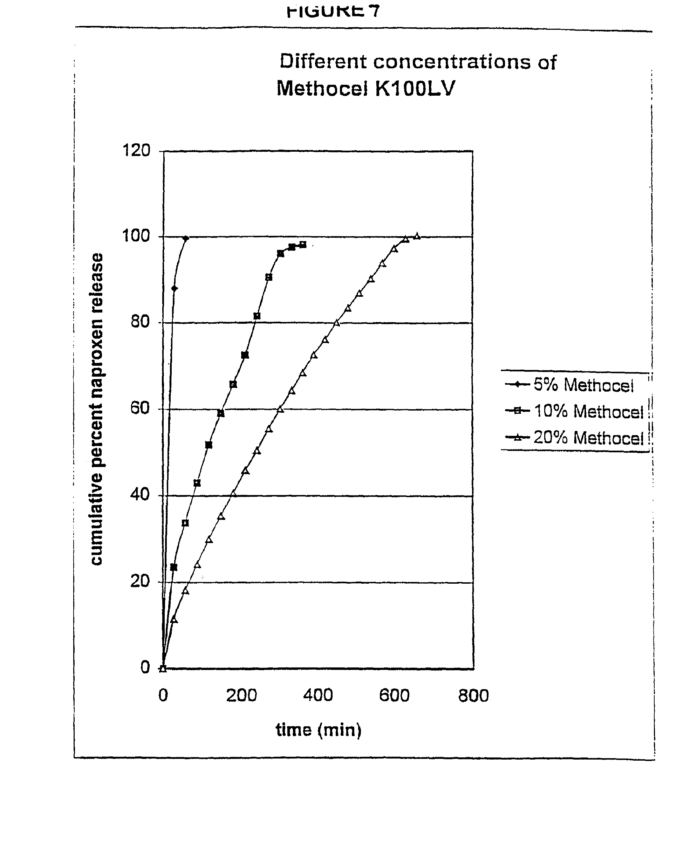

The results, shown in Figure 7, show that with tablets having an identical hardness and varying rate-controlling polymer concentrations, the tablet having the greatest rate-controlling polymer concentration will have the most prolonged drug release characteristics. The tablet having a 5% polymer concentration completely released after about 50 min.; the tablet having a 10% polymer concentration completely released after about 350 min.; and the tablet having a 20% polymer concentration completely released after about 650 min. Thus, increased polymer concentration in the nanoparticulate formulation is directly correlated with prolonged release of the administered agent.

### Example 9

The purpose of this example was to determine the effect of wet granulation on controlled release of nanoparticulate formulations.

Tablets were formed as in Example 1, except that a small amount of water was added into each mixture to form granules. The granules were then sieved through a coarse mesh screen and dried. The material was then re-sieved through a fine mesh screen, and blended with magnesium stearate and lactose, followed by tableting to create tablets. Quantities of each component in each of the tablet formulations are given below (mg).

| **Naproxen SDI** | **Klucel^{®} HPC** | **Methocel^{®} HPMC** | **Lactose** | **Mg Stearate** |
|---|---|---|---|---|
| 292.5 | 150 | 0 | 300 | 7.5 |
| 292.5 | 0 | 150 | 300 | 7.5 |

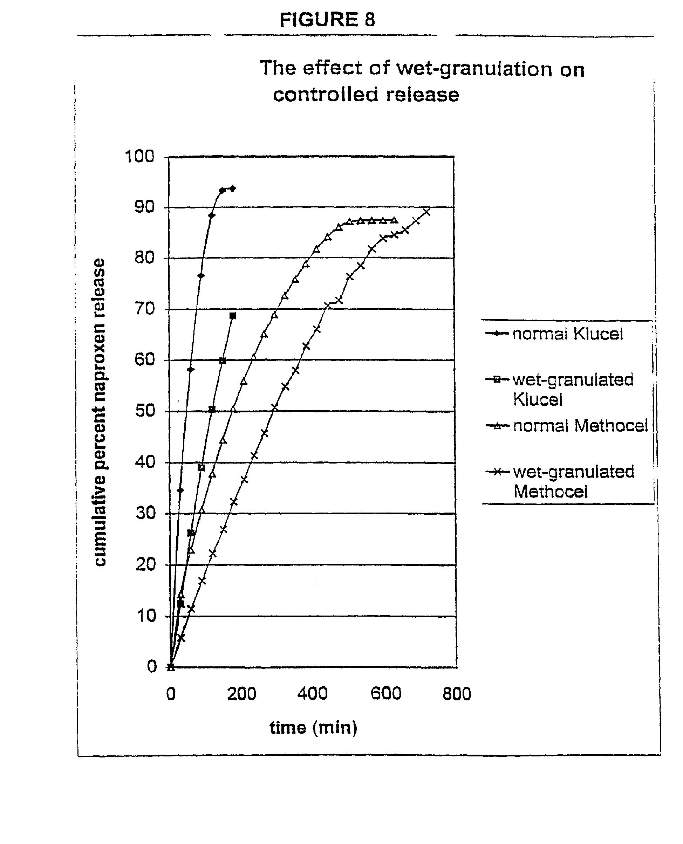

The results, shown in Figure 8, indicate that for both rate-controlling polymers, KIucel^{®} HPC and Methocel^{®} HPMC, the tablets formed from wet granulation showed a much more controlled release than the normal dry mixture. The prolonged controlled release is likely due to the strong binding of the granules formed by the wet granulation technique. This binding is stronger than the binding of the materials by direct compression. Thus, wet granulation improves controlled release.

### Example 10

The purpose of this example was to prepare a controlled release formulation of glipizide. Glipizide, also known as 1-cyclohexyl-3[[*p*-[21(5-methylpyrazine-carboxyamido)ethyl]-phenyl]-sulfonyl]-urea, is an oral sulfonylurea.

Glipizide and HPC-SL in the ratio of 10:3, were milled in a Dyno-mill to produce a nanoparticulate glipizide dispersion. The composition was milled for 6 hours, and the average effective particle size of the glipizde was about 177 nm, with about 50% of the particles having a size less than about 157 nm, and about 90% of the particles having a size less than about 276 nm.

The nanoparticulate glipizide suspension was spray dried using a Yamato GB-22^{®} spray-dryer under following conditions to produce a spray-dried glipizide intermediate (SDI):

| | |
|---|---|
| Inlet temp.: | 115°C |
| Outlet temp.: | 50°C |
| drying air | 0.36 m³/min |
| atomizing air | 2.5 Kgf/cm² |

The powder blend for the tablets comprised: 13 mg SDI, 241.6 mg Methocel^{®} (K100LV), 483.3 mg lactose (Foremost # 316), and 12.1 mg magnesium stearate, for a total of 750.0 mg. Each 750.0 mg tablet contained 10 mg of the drug (glipizide)

The excipients were sieved, blended, and compressed using a Carver press at 5,000 1b for 10 sec. The tablets were analyzed (at 274 nm) using the dissolution system as described above.

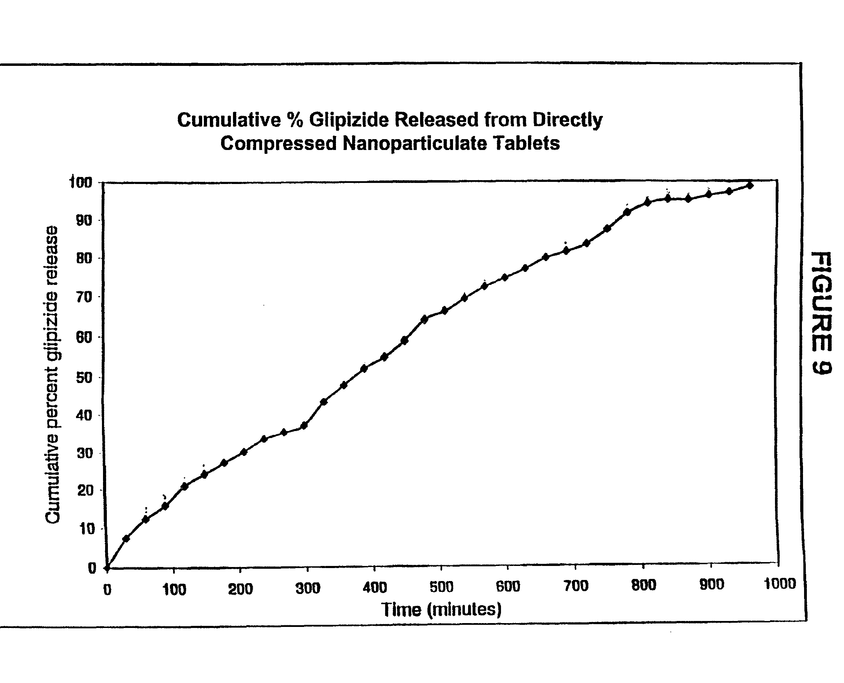

The results, shown in Figure 9, indicate a steady release of drug over a time period of just under 16 hours (i.e., about 950 minutes).

In Examples 11-15, all percentages are by weight unless otherwise stated. The term "purified water" refers to water which has been passed through a water filtration system.

### Example 11

The purpose of this example was to prepare an uncoated controlled release tablet formulation containing nanoparticulate nifedipine.

A colloidal dispersion of nifedipine in water was prepared. The dispersion contained 10 % (w/w) of the drug and 2 % hydroxypropyl cellulose. Particle size analysis, performed using a Malvern Mastersizer S2.14 (Malvern Instruments Ltd., Malvern, Worcestershire, UK) recorded by a wet method using a 150 ml flow through cell, revealed the following particle size characteristics: D_{v,90} 620 nm; D_{v,50} 313nm; D_{v,10} 170 nm, with 97.47 % of the colloidal particles being less than 1.03 µm in diameter. (Where D_{v,90} 620 nm indicates that 90 % of particles had a size less than 620 nm, etc.).

The nifedipine dispersion was prepared for spray drying by a series of four homogenization steps. The dispersion was homogenized at medium shear for 5 min. Sodium lauryl sulphate (0.05 %) was added prior to homogenization at medium shear for a further 5 min. The dispersion was then diluted 50:50 with purified water and homogenized at medium shear for a further 10 min. Finally, mannitol (10 %) was added and the mixture was homogenized at high shear for 15 min. The final content of the mixture to be spray dried is given in Table 1.

| Table 1 Composition prior to spray drying for Example 11 | |
|---|---|
| Ingredient | Amount (% by wt.) |
| Nifedipine dispersion | 45.44 |
| Purified water | 45.44 |
| Mannitol | 9.09 |
| Sodium lauryl sulphate | 0.02 |

The mixture thus obtained was spray dried using a Büchi Mini B-191 Spray Drier system (Büchi, Switzerland). The spray drying conditions are summarized in Table 2. The spray dried nifedipine particles thus prepared were then blended. The blend formulation is given in Table 3.

| Table 2 Spray drying conditions for Example 11 | |
|---|---|
| Parameter | Level |
| Inlet temperature | 135°C |
| Atomising pressure setting | 800 l/min |
| Vacuum pressure | 30-45 mbar |
| Aspirator setting | 100 % |
| Spray rate | 6 ml/min |

The blend obtained after the previous step was tableted manually using a Fette E1 tablet press (Wilheim Fette GmbH, Schwarzembek, Germany) fitted with 11 mm round normal concave tooling. The tablets produced had a mean tablet hardness of 122.7 N and a mean tablet potency of 29.7 mg/ tablet. *In vitro* dissolution was carried out in phosphate - citrate buffer, pH 6.8, containing 0.5 % sodium lauryl sulphate, using USP apparatus II (100 rpm). Dissolution data is given in Table 4.

| Table 3 Blend formulation for Example 11 | |
|---|---|
| Ingredient | Amount |
| Spray dried nifedipine | 17.92 |
| Avicel PH102 | 30.01 |
| Pharmatose DCL | 30.01 |
| Methocel K 15M | 20.00 |
| Colloidal silicon dioxide | 1.20 |
| Magnesium stearate | 0.86 |

| Table 4 Dissolution data for uncoated nifedipine tablets prepared according to Example 11 | |
|---|---|
| Time (hr) | % Active Released |
| 1.0 | 17.8 |
| 2.0 | 24.9 |
| 4.0 | 37.1 |
| 6.0 | 49.1 |
| 8.0 | 61.5 |
| 10.0 | 71.5 |
| 22.0 | 108.8 |

### Example 12

The purpose of this example was to prepare a coated controlled release tablet formulation containing nanoparticulate nifedipine.

Tablets prepared according to Example 11 were coated with a Eudragit^{®} L coating solution detailed in Table 5. Coating was performed using an Manesty Accelacota 10" apparatus (Manesty Machine Ltd., Liverpool, UK) and a coating level of 5.5 % solids weight gain was achieved. Coating conditions are given in Table 6.

| Table 5 Coating solution formulation | |
|---|---|
| Ingredient | Amount (%) |
| Eudragit^{®} L 12.5 | 49.80 |
| Talc | 2.49 |
| Dibutyl sebecate | 1.25 |
| Isopropyl alcohol | 43.46 |
| Purified water | 3.00 |

| Table 6 Coating conditions | |
|---|---|
| Parameter | Level |
| Inlet temperature | 35 - 45 °C |
| Outlet temperature | 32 - 36 °C |
| Air pressure | 1.4 bar |
| Spray rate | 27 g/min |

*In vitro* dissolution was carried out according to the same methodology used in Example 1: phosphate - citrate buffer, pH 6.8, containing 0.5 % sodium lauryl sulphate, using USP apparatus II (100 rpm). Dissolution data is given in Table 7.

| Table 7 Dissolution data for coated nifedipine tablets prepared according to Example 12 | |
|---|---|
| Time (hr) | % Active Released |
| 1.0 | 4.3 |
| 2.0 | 11.5 |
| 4.0 | 24.0 |
| 6.0 | 38.0 |
| 8.0 | 58.3 |
| 10.0 | 66.4 |
| 22.0 | 99.6 |

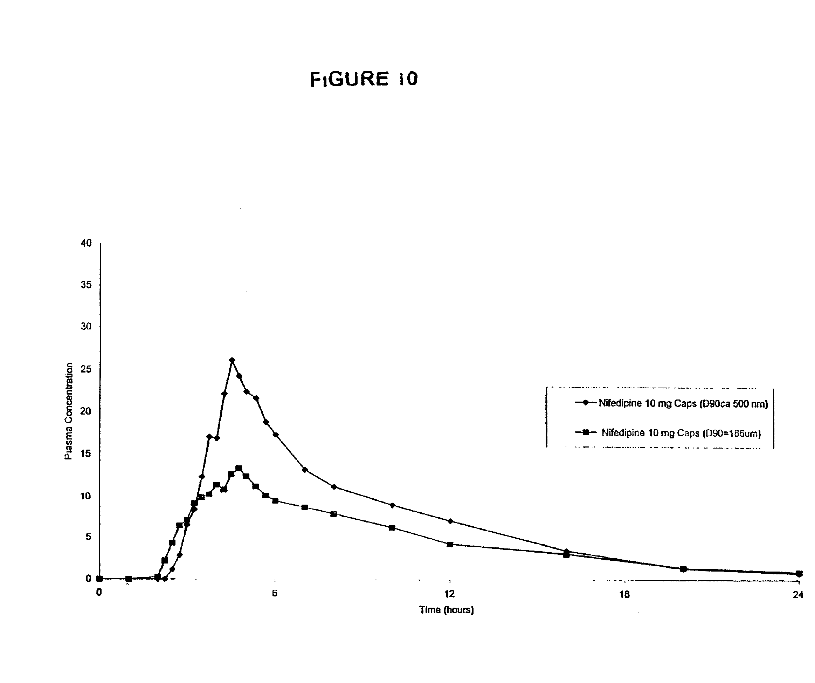

Figure 10 shows the mean *in vivo* plasma profiles in nine fasted human volunteers for (1) nifedipine containing controlled release matrix tablets coated with a controlled release coating according to the present invention as described in Example 12; and (2) a control composition. The study had a fully randomized, fully crossed over, single dose administration design. From the figure it can be seen that a controlled release composition prepared according to Example 12 shows a high level of availability and shows good controlled release characteristics over a 24 hour period.

### Example 13

The purpose of this example was to prepare an uncoated controlled release tablet formulation containing nanoparticulate glipizide.

A colloidal dispersion of glipizide in water was prepared. The dispersion contained 10 % (w/w) of the drug and 3 % hydroxypropyl cellulose. Particle size analysis, performed using a Malvern Mastersizer S2.14, recorded by a wet method using a 150 ml flow through cell, revealed the following particle size characteristics: D_{v,90} 650 nm; D_{v,50} 386 nm; D_{v,10} 290 nm.
The glipizide dispersion was prepared for spray drying by adding 15 % mannitol to the aqueous glipizide dispersion with stirring. The final content of the mixture to be spray dried is given in Table 8.

| Table 8 Composition prior to spray drying for Example 13 | |
|---|---|
| Ingredient | Amount (% by wt.) |
| Glipizide dispersion | 10 |
| Hydroxypropyl cellulose | 3 |
| Mannitol | 15 |
| Purified water | 72 |

The mixture thus obtained was spray dried using a Büchi Mini B-191 Spray Drier system. The spray drying condition are summarized in Table 9.

| Table 9 Spray drying conditions for Example 13 | |
|---|---|
| Parameter | Level |
| Inlet temperature | 115-116 °C |
| Atomising pressure setting | 800 mbar |
| Vacuum pressure | 25-45 mbar |
| Aspirator setting | 100 % |
| Spray rate | 10 ml/min |

The spray dried glipizide particles thus prepared were then blended. The blend formulation is given in Table 10.

| Table 10 Blend formulation for Example 13 | |
|---|---|
| Ingredient | Amount (% by wt.) |
| Spray dried glipizide | 3.36 |
| Avicel^{™} pH101 | 35.8 |
| Methocel K^{™} 100LV | 60.0 |
| Aerosil^{™} 200 | 0.4 |
| Magnesium stearate | 0.5 |

The blend obtained after the previous step was tableted using a single station tablet press fitted with 9.5 mm round normal concave tooling. The tablets produced had a mean tablet hardness of 149 N and a mean tablet potency of 9.1 mg/ tablet. *In vitro* dissolution was carried out in KH₂PO₄ buffer, pH 7.5, using USP apparatus I (100 rpm). Dissolution data is given in Table 11.

| Table 11 Dissolution data for uncoated glipizide tablets prepared according to Example 13 | |
|---|---|
| Time (hr) | % Active Released |
| 1.0 | 8.0 |
| 2.0 | 17.0 |
| 4.0 | 35.1 |
| 6.0 | 51.4 |
| 8.0 | 65.2 |
| 10.0 | 79.5 |
| 22.0 | 95.6 |

### Example 14

The purpose of this example was to prepare delayed release nanoparticulate nifedipine capsules.

A colloidal dispersion of nifedipine in water was prepared. The dispersion contained 10% w/w Nifedipine, 2% hydroxypropylcellulose, and 0.1 % Sodium Lauryl Sulphate in water. Particle size analysis, performed using a Malvern Mastersizer S2.14, recorded by a wet method using a 150 ml flow through cell, revealed the following particle size characteristics: Dv,90 = 490 nm; Dv,50 = 290 nm; Dv,10 = 170 nm

The nifedipine dispersion was prepared for spray drying by adding Purified Water and homogenizing for 5 minutes. Mannitol was added and the resulting mixture was homogenized for 15 minutes. The final content of the mixture to be spray dried is given in Table 12.

| Table 12 Composition prior to spray drying for Example 14 | |
|---|---|
| Ingredient | Amount (% by wt.) |
| Nifedipine dispersion | 45.45 |
| Mannitol | 9.09 |
| Purified water | 45.45 |

The mixture thus obtained was spray dried using a Buchi Mini B-191 Spray Drier system. The spray drying conditions are summarized in Table 13.

| Table 13 Spray drying conditions for Example 14 | |
|---|---|
| Parameter | Level |
| Inlet temperature | 135°C |
| Atomising pressure setting | 800 mbar |
| Aspirator setting | 100 % |
| Flow rate | 6 ml/min |

The spray dried nifedipine particles thus prepared were then blended. The blend formulation is given in Table 14.

| Table 14 Blend formulation for Example 14 | |
|---|---|
| Ingredient | Amount (% by wt.) |
| Spray dried nifedipine (Dv,90 *ca* 500 nm) | 10.40 |
| Avicel^{™} pH102 | 77.05 |
| Explotab | 10.00 |
| Colloidal Silicon Dioxide | 1.00 |
| Magnesium stearate | 1.50 |

The resulting blend was tableted using a Fette P2100 rotary tablet press (Wilhelm Fette GmbH, Schwarzenbek, Germany) fitted with 3.8 mm shallow concave multi-tipped tooling. The tablets had a mean set up hardness of 56 N and a mean set up weight of 34.46 mg.

The tablets thus obtained were coated in a Hi-Coater (Vector Corp., Marion, IA, USA) with the Eudragit S coating solution detailed in Table 15. A coating level of 10.03 % solids weight gain was achieved.

| Table 15 Coating Solution Formulation for Example 14 | |
|---|---|
| Ingredient | Amount (% by wt.) |
| Eudragit S 12.5 | 50.0 |
| Talc | 2.50 |
| Dibutyl Sebecate | 1.25 |
| Isopropyl Alcohol | 43.25 |
| Purified Water | 3.00 |

The coated minitablets thus obtained were hand-filled into hard gelatin capsules to form Nifedipine 10 mg Capsules ( 9 minitablets / capsule). *In vitro* dissolution was carried out in citrate-phosphate buffer, pH 6.8, containing 0.5 % Sodium Lauryl Sulphate, using a USP apparatus II (100 rpm). The dissolution data of the resulting capsules is given in Table 16.

| Table 16 Dissolution data for Nifedipine 10 mg capsules prepared according to Example 14 | |
|---|---|
| Time (hr) | % Active Released |
| 0.25 | 3.99 |
| 0.5 | 4.60 |
| 0.75 | 21.10 |
| 1.0 | 93.07 |
| 1.5 | 100.39 |
| 2.0 | 100.79 |

### Example 15

The purpose of this example was to prepare a control for delayed release nanoparticulate nifedipine capsules. The control does not contain a nanoparticulate composition.

Nifedipine raw material (Dv, 90 = 673 µm), Explotab, and Avicel pH 102 were mixed in the Gral 25 (NV-Machines Colett SA, Wommelgam, Belgium) for 10 minutes at 1000 rpm. Purified water was gradually added with mixing until granulation was achieved. The granulate was oven dried for 18 hours at 50 °C. The dried granulate was milled through a 50 mesh screen using a Fitzmill M5A (The Fitzpatrick Co. Europe, Sint-Niklaas, Belgium). The final content of the granulate is summarized in Table 17.

| Table 17 Final composition of Granulate for Example 15 | |
|---|---|
| Ingredient | Amount (% by wt.) |
| Nifedipine | 7.68 |
| Explotab | 24.22 |
| Avicel pH 102 | 68.10 |

The granulate thus obtained (Dv, 90 = 186 µm) was then blended. The blend formulation is given in Table 18.

| Table 18 Blend Formulation for Example 15 | |
|---|---|
| Ingredient | Amount (% by wt.) |
| Nifedipine Granulate (Dv, 90 =186 µm) | 41.28 |
| Avicel pH102 | 56.22 |
| Colloidal Silicon Dioxide | 1.00 |
| Magnesium Stearate | 1.50 |

The particle size analysis of the starting nifedipine raw material and the milled nifedipine granulate, performed using the Malvern Mastersizer S with a 1000 mm lens (nifedipine raw material) and a 300 mm lens (milled nifedipine granulate) recorded by a dry powder method, revealed the particle size characteristics given in Table 19.

| Table 19 Particle Size Analysis of Nifedipine Compositions | | |
|---|---|---|
| Size Range | Raw Nifedipine | Milled Nifedipine Granulate |
| Dv, 90 | 673 µm | 186 µm |
| Dv, 50 | 234 µm | 103 µm |
| Dv, 10 | 14 µm | 32 µm |

The resulting blend was tableted using a Fette P2100 rotary tablet press fitted with 3.8 mm shallow concave multi-tipped tooling. The tablets had a mean set up hardness of 47 N and a mean set up weight of 35 mg. The tablets thus obtained were coated in a Hi-Coater with the Eudragit S coating solution detailed in Table 20. A coating level of 10.34 % solids weight gain was achieved.

| Table 20 Coating Solution Formulation for Example 15 | |
|---|---|
| Ingredient | Amount (% by wt.) |
| Eudragit S 12.5 | 50.0 |
| Talc | 2.50 |
| Dibutyl Sebecate | 1.25 |
| Isopropyl Alcohol | 43.25 |
| Purified Water | 3.00 |

The coated minitablets thus obtained were hand-filled into hard gelatin capsules to form nifedipine 10 mg capsules (9 minitablets/capsule). *In vitro* dissolution was carried out in citrate-phosphate buffer, pH 6.8, containing 0.5 % Sodium Lauryl Sulphate, using USP apparatus II (100 rpm). The dissolution data for the resulting capsules is given in Table 21.

| Table 21 Dissolution data for Nifedipine 10 mg capsules prepared according to Example 15 | |
|---|---|
| Time (hr) | % Active Released |
| 0.25 | 8.83 |
| 0.5 | 32.50 |
| 0.75 | 77.88 |
| 1.0 | 85.26 |
| 1.5 | 91.30 |
| 2.0 | 94.46 |

### Example 16

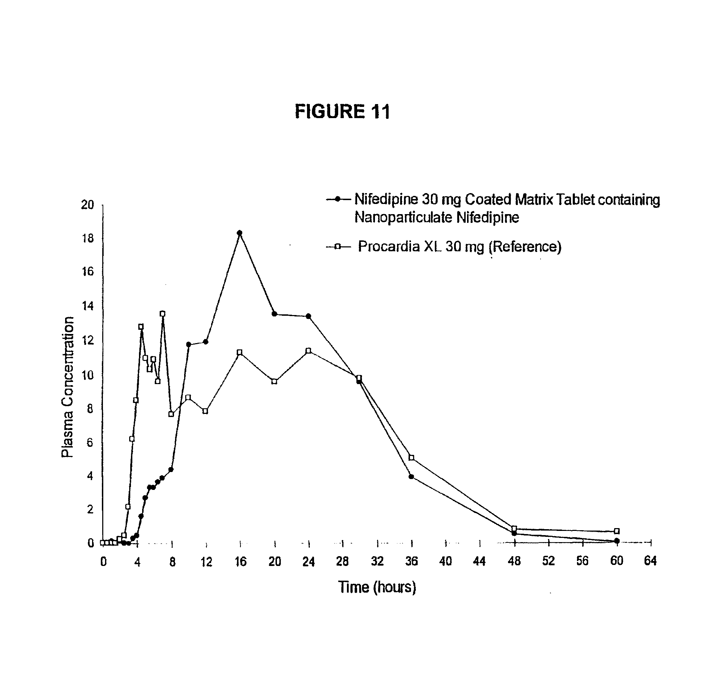

Figure 11 shows the mean *in-vivo* plasma profiles of nifedipine in ten fasted human volunteers for: (1) a controlled release composition manufactured according to the present invention as described in Example 14 (nifedipine 10 mg capsules (Dv, 90 *ca* 500 nm)); and (2) a control composition manufactured as described in Example 15 (nifedipine 10 mg capsules (Dv,90 = 186 µm)). The study had a single dose, fully randomized, fully crossed over, oral administration design. From the Figure it can be seen that the controlled release composition manufactured according to the present invention shows an initial lag time followed by a rapid and high level of availability of active.

It should be noted that the controlled release composition manufactured in accordance with the invention showed a relative bioavailability of 1.45 *(i.e.,* 45% enhanced bioavailability as compared with the control).

### Example 17

The purpose of this experiment was to determine the effects of the polymers Carbopol^{®} and Eudragit^{®} on controlled release of directly compressed nanoparticulate naproxen tablets. Carbopols^{®} are cross-linked polyacrylate materials available from BF Goodrich, Cleveland, OH, and Eudragits^{®} are methacrylic-acrylic copolymers available from Rohm Pharma, Darmstadt, Germany.

Four specific polymer types and grades were tested: Carbopol^{®} grade 971P NF, Carbopol^{®} grade 974P-NF, Eudragit^{®} grade RS PO, and Eudragit^{®} grade RL PO. Prior to use, each polymer was sieved through #40 mesh.

The tablets were prepared by first sieving the spray-dried drug intermediate (SDI), the Carbopol^{®} or Eudragit^{®} polymer, and excipients, listed below, through a #40 mesh:
(1) The SDI comprised 93% naproxen and 7% polyvinylpyrrolidone;
(2) lactose as an excipient (NF lactose monohydrate, Fast-Flo, Foremost Farms U.S.A.); and
(3) magnesium stearate as an excipient (Spectrum).

To prepare the tablets for dissolution, material blends were formulated. After the SDI and each excipient were initially sieved, each was added in proper amounts to the V-blender (Blend Master Lab Blender, Patterson Kelley Co.). After blending, formulations were pressed into 750 mg tablets through use of the automated Carver Press (Carver, Inc., Wabash Indiana).

Three tablets were tested simultaneously in the Distek Dissolution System for each individual formulation at specific parameters. The Distek Dissolution System contains a water bath heated to 37°C as well as vessels containing phosphate buffer heated to 37°C. 900 ml of buffer were inserted into three or six different vessels to test three or six tablets at one time. A paddle was placed into each vessel where it rotated at 50 rpm. As the tablets dissolved, a small pump drew buffer and drug from the vessels to analyze them to determine the amount of drug released.

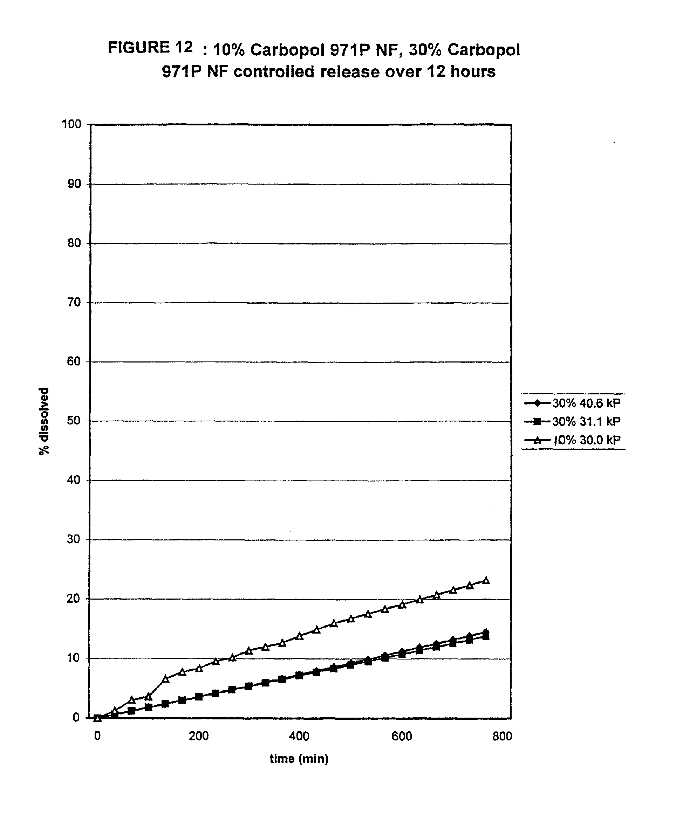

Figures 12-14 show release profiles of each polymer at different levels of hardness and concentration. Figure 12 shows the controlled release profile of tablets containing 30% Carbopol^{®} 971P NF polymer. The tablets in this test were compressed to both 40.6 kP and 31.1 kP. Under these conditions, Carbopol^{®} 971 P NF allowed for very little drug release over a period of 12 hours, as shown in Figure 12. Approximately 14-15% of the naproxen drug was released. At this rate, the entire tablet would require between 85 and 90 hours to completely dissolve. The difference in hardness (40.6 and 31.1 kP) made little to no considerable difference with the polymer concentration as high as 30%.

Figure 12 also shows the results of tablets having 10% Carbopol^{®} 971P NF. The hardness of this tablet was 30.0 kP. A total of 22% of the drug was released over a period of 12 hours. This shows a mild increase in the rate of drug release for tablets comprising 10% Carbopol^{®} as compared to tablets comprising 30% Carbopol^{®}. Regardless of the concentration of Carbopol^{®}, tablets comprising Carbopol^{®} polymers exhibit a higher level of controlled release with an increased tablet hardness.

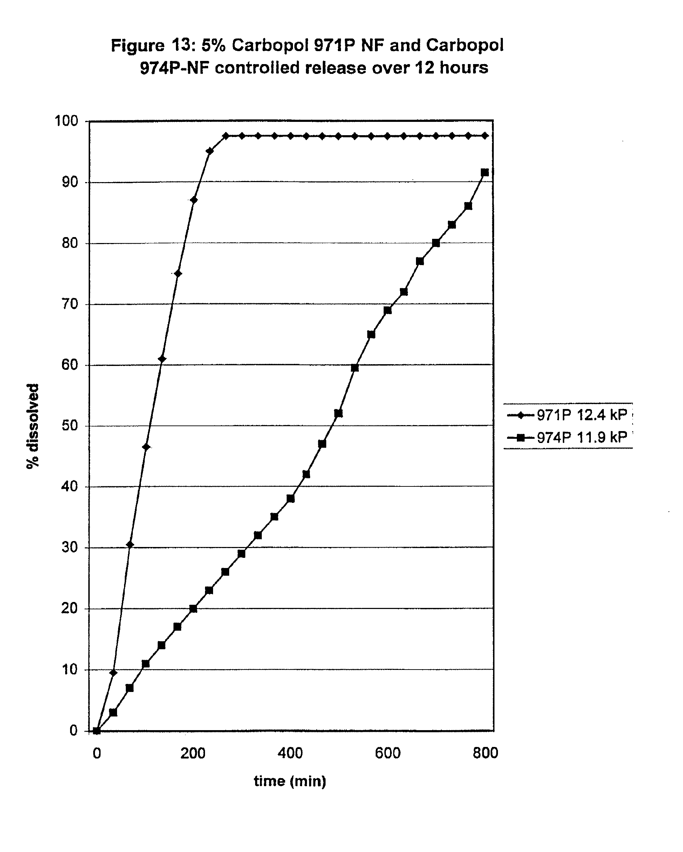

Figure 13 shows a comparison between Carbopol^{®} 971P NF and Carbopol^{®} 974P-NF at a 5% percent concentration with relatively low hardness (12.4 and 11.9 kP, respectively). Carbopol^{®} 974P-NF produced a drug release that was nearly three times the speed of Carbopol^{®} 971P NF. Under these specific conditions, Carbopol^{®} 971P NF allowed for almost zero-order 100% release in twelve hours.

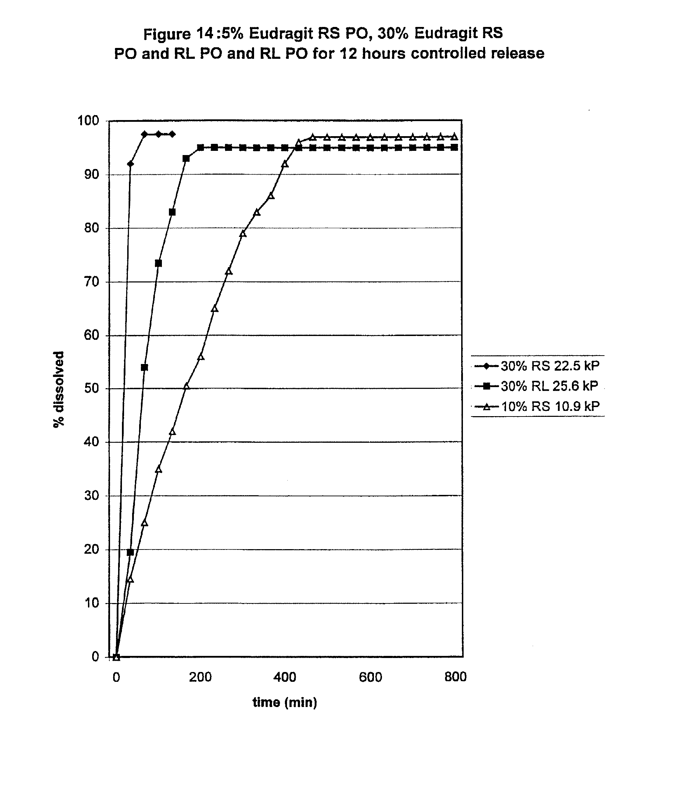

Figure 14 shows the effect of the polymer Eudragit^{®} grades RS PO and RL PO on controlled release. This polymer actually showed a trend indicating a more immediate release. With a 5% concentration at low hardness (10.9 kP), the Eudragit^{®} RS PO polymer allowed for the tablet to dissolve in only one hour. These grades were tested at a concentration of 30% and at a hardness of between 22 and 27 kP. Eudragit^{®} RL PO under these conditions acted faster than Eudragit^{®} RS PO, releasing all of the naproxen within the time frame of three hours. On the other hand, Eudragit^{®} RS PO under these conditions allowed for 100% release within six to seven hours.

Carbopol^{®} 971P NF and Carbopol^{®} 974P-NF allowed for the greatest level of controlled release. At high concentration and high hardness, this polymer released all of the naproxen over a period of a few days (nearly 90 hours). However at low concentration and low hardness, the grade 971P NF allowed for a near zero release profile over 13-14 hours. Eudragit^{®} RS PO and Eudragit^{®} RL PO produced a different profile compared to the Carbopol^{®} grades. The Eudragit^{®} polymers allowed for a more immediate release of drug at low hardness and concentration (1-2 hours) and a slightly slower release at high concentration and hardness (6-7 hours). Optimal controlled release profiles were found at low concentration and hardness of the Carbopol^{®} polymer and high concentration and hardness of the Eudragit^{®} polymer.

It will be apparent to those skilled in the art that various modifications and variations can be made in the methods and compositions of the present invention without departing from the spirit or scope of the invention. Thus, it is intended that the present invention cover the modifications and variations of this invention provided they come within the scope of the appended claims and their equivalents.

In view of the foregoing, it will be appreciated that the invention described herein *inter alia* relates to the following items:
1. A controlled release nanoparticulate composition comprising:
   (a) a poorly soluble agent to be administered having an effective average particle size of less than about 1000 nm;
   (b) at least one surface stabilizer associated with the surface of the agent, and
   (c) at least one pharmaceutically acceptable rate-controlling polymer,
   wherein the composition provides controlled release of the agent for a time period ranging from about 2 to about 24 hours or longer.
2. The composition of item 1, wherein the effective average particle size of the agent is selected from the group consisting of less than about 800 nm, less than about 600 nm, less than about 400 nm, less than about 300 nm, less than about 250 nm, less than about 100 nm, and less than about 50 nm.
3. The composition of item 1, wherein the concentration of the polymer is from about 5 to about 95% (w/w).
4. The composition of item 3, wherein the concentration of the polymer is from about 10 to about 65% (w/w).
5. The composition of item 1 additionally comprising a binder agent in an amount of from about 0.1 to about 10% (w/w).
6. The composition of item 1 additionally comprising a lubricant in an amount of from about 0.1 to about 10% (w/w).
7. The composition of item 6, wherein the lubricant is selected from the group consisting of magnesium stearate, hydrogenated vegetable oil, and stearic acid.
8. The composition of item 1, wherein the solid dose formulation is made by wet granulation.
9. The composition of item 1 formed by wet granulation, wherein water is added to the agent, surface stabilizer, and polymer to form granules prior to forming the solid dose of the controlled release formulation.
10. The composition of item 1, wherein the rate-controlling polymer is selected from the group consisting of gum arabic, agar, guar gum, cereal gums, dextran, casein, gelatin, pectin, carrageenan, waxes, shellac, hydrogenated vegetable oils, polyvinylpyrrolidone, hydroxypropyl cellulose (HPC), hydroxyethyl cellulose (HEC), hydroxypropyl methylcelluose (HPMC), sodium carboxymethylcellulose (CMC), poly(ethylene) oxide, alkyl cellulose, ethyl cellulose, methyl cellulose, carboxymethyl cellulose, hydrophilic cellulose derivatives, polyethylene glycol, polyvinylpyrrolidone, cellulose acetate, cellulose acetate butyrate, cellulose acetate phthalate, cellulose acetate trimellitate, polyvinyl acetate phthalate, hydroxypropylmethyl cellulose phthalate, hydroxypropylmethyl cellulose acetate succinate, polyvinyl acetaldiethylamino acetate, poly(alkylmethacrylate), poly(vinyl acetate), polymers derived from acrylic or methacrylic acid and their respective esters, and copolymers derived from acrylic or methacrylic acid and their respective esters.
11. The composition of item 10, wherein the rate-controlling polymer is HPMC.
12. The composition of item 10, wherein the rate-controlling polymer is a polymer derived from acrylic or methacrylic acid and their respective esters or copolymers derived from acrylic or methacrylic acid and their respective esters.
13. The composition of item 1, wherein the poorly water soluble agent is present in an amount of from about 1 µg to about 800 mg.
14. A dosage form comprising a controlled release nanoparticulate composition according to item 1 , wherein the dosage form is in tablet form or in multiparticulate form.
15. The dosage form of item 14, wherein the agent and at least one auxilary excipient are compressed into tablet form prior to coating with a rate controlling polymer.
16. The dosage form of item 14, wherein the agent, the rate controlling polymer and at least one auxilary excipient are compressed to form a controlled release matrix tablet.
17. The dosage form of item 16, wherein the controlled release matrix is coated with a rate controlling polymer.
18. The dosage form of item 14, wherein the agent and at least one auxilary excipient are compressed into the form of a multilayer tablet prior to coating with a rate controlling polymer.
19. The dosage form of item 14, wherein the agent is dispersed in the rate controlling polymer material and compressed into the form of a multilayer tablet.
20. The dosage form of item 19, wherein the multilayer tablet is coated with a rate controlling polymer.
21. The dosage form according to item 14, wherein the agent, at least one auxiliary excipient, and the rate controlling polymer material are combined into a multiparticulate form.
22. The dosage form according to item 21, wherein the multiparticulate form comprises discrete particles, pellets, minitablets, or combinations thereof.
23. The dosage form according to item 21, wherein the multiparticulate is encapsulated in hard or soft gelatin capsules.
24. The dosage form according to item 21 wherein the multiparticulate is incorporated into a sachet.
25. The dosage form according to item 22 wherein the discrete particles or pellets are compressed into tablet form.
26. The dosage form according to item 25 wherein the tablet form is coated with a rate controlling polymer material.
27. The dosage form according to item 22 wherein the discrete particles or pellets are compressed into a multilayer tablet.
28. The dosage form according to item 27 wherein the multilayer tablet is coated with a rate controlling material.
29. The dosage form according to item 14 wherein the tablet further comprises an osmagent added to the controlled release composition to form an admixture and a semi-permeable membrane; the semi-permeable membrane surrounding the admixture and being permeable to aqueous media, but impermeable to the poorly soluble drug compound or pharmaceutically acceptable salt thereof and the semi-permeable membrane defining an orifice therein.
30. A method of preparing a solid dose controlled release nanoparticulate formulation comprising:
   (a) combining a nanoparticulate composition of an agent to be administered and at least one surface stabilizer associated with the surface of the agent, wherein the composition has an effective average particle size of less than about 1000 nm and at least one suitable rate-controlling polymer; and
   (b) forming a solid dose of the mixture from step (a), wherein the solid dose formulation has a controlled release of the agent following administration for a time period ranging from about 2 to about 24 hours or longer.
31. The method of item 30, wherein the effective average particle size of the agent particles is selected from the group consisting of less than about 800 nm, less than about 600 nm, less than about 400 nm, less than about 300 nm, less than about 250 nm, less than about 100 nm, and less than about 50 nm.
32. The method of item 30, wherein the concentration of the polymer is from about 5 to about 95% (w/w).
33. The method of item 32, wherein the concentration of the polymer is from about 10 to about 65% (w/w).
34. The method of item 31, comprising adding water to the nanoparticulate agent, surface stabilizer, and rate-controlling polymer to form granules prior to step (b).
35. A method of treating a mammal comprising administering to the mammal an effective amount of a solid dose controlled release nanoparticulate formulation wherein:
   (a) the formulation comprises nanoparticulate agent particles to be administered and at least one surface stabilizer associated with the surface of the nanoparticulate agent, wherein the agent particles have an effective average particle size of less than about 1000 nm and at least one suitable rate-controlling polymer; and
   (b) the formulation has a controlled release of the agent following administration for a time period ranging from about 2 to about 24 hours or longer.

## Claims

1. A solid dose controlled release composition comprising:
a redispersible drug material consisting of a spray dried dispersion of the drug;
a rate controlling polymer; and
one or more excipients;
wherein the nanoparticulate drug material has
(i) at least one surface stabilizer associated with the surface of the drug particles, and
(ii) an effective particle size of less than 1000 nm
and wherein the controlled release composition provides controlled release of the nanoparticlate drug for a time period of from 2 to 24 hours or longer.

2. The composition of claim 1, wherein the effective average particle size of the drug is selected from the group consisting of less than 800 nm, less than 600 nm, less than 400 nm, less than 300 nm, less than 250 nm, less than 100 nm, and less than 50 nm.

3. The composition of claim 1, wherein the concentration of the polymer is from 5 to 95% (w/w).

4. A method of preparing a solid dose controlled release composition by wet granulation comprising:
(a) combining a nanoparticulate composition of a drug and at least one surface stabilizer associated with the surface of the drug, wherein the composition has an effective average particle size of less than about 1000 nm and;
(b) integrating at least one suitable rate-controlling polymer in a rate-controlling matrix with the nanoparticulate drug composition or coating the nanoparticulate drug composition with a rate-controlling polymer; and
(c) mixing water and/or granulating fluid to the drug, surface stabilizer, and polymer to form granules.

5. The composition of claim 1, wherein the rate-controlling polymer is selected from the group consisting of gum arabic, agar, guar gum, cereal gums, dextran, casein, gelatin, pectin, carrageenan, waxes, shellac, hydrogenated vegetable oils, polyvinylpyrrolidone, hydroxypropyl cellulose (HPC), hydroxyethyl cellulose (HEC), hydroxypropyl methylcelluose (HPMC), sodium carboxymethylcellulose (CMC), poly(ethylene) oxide, alkyl cellulose, ethyl cellulose, methyl cellulose, carboxymethyl cellulose, hydrophilic cellulose derivatives, polyethylene glycol, polyvinylpyrrolidone, cellulose acetate, cellulose acetate butyrate, cellulose acetate phthalate, cellulose acetate trimellitate, polyvinyl acetate phthalate, hydroxypropylmethyl cellulose phthalate, hydroxypropylmethyl cellulose acetate succinate, polyvinyl acetaldiethylamino acetate, poly(alkylmethacrylate), poly(vinyl acetate), polymers derived from acrylic or methacrylic acid and their respective esters, and copolymers derived from acrylic or methacrylic acid and their respective esters.

6. The composition of claim 5, wherein the rate-controlling polymer is HPMC.

7. The composition of claim 5, wherein the rate-controlling polymer is a polymer derived from acrylic or methacrylic acid and their respective esters or copolymers derived from acrylic or methacrylic acid and their respective esters.

8. A dosage form comprising a controlled release nanoparticulate composition according to claim 1, wherein the dosage form is in tablet form or in multiparticulate form.

9. The dosage form of claim 8, wherein the drug and at least one auxiliary excipient are compressed into tablet form prior to coating with a rate controlling polymer.

10. The dosage form of claim 8, wherein the drug, the rate controlling polymer and at least one auxiliary excipient are compressed to form a controlled release matrix tablet.

11. The dosage form of claim 10, wherein the controlled release matrix is coated with a rate controlling polymer.

12. The dosage form according to claim 8, wherein the drug, at least one auxiliary excipient, and the rate controlling polymer material are combined into a multiparticulate form.

13. The dosage form according to claim 12, wherein the multiparticulate form comprises discrete particles, pellets, minitablets, or combinations thereof.

14. The dosage form according to claim 13 wherein the discrete particles or pellets are compressed into tablet form.

15. The dosage form according to claim 14 wherein the tablet form is coated with a rate controlling polymer material.

16. The dosage form according to claim 14 wherein the tablet further comprises an osmagent added to the controlled release composition to form an admixture and a semi-permeable membrane; the semi-permeable membrane surrounding the admixture and being permeable to aqueous media, but impermeable to the poorly soluble drug compound or pharmaceutically acceptable salt thereof and the semi-permeable membrane defining an orifice therein.
